# EUROPEAN PATENT SPECIFICATION

(11) **EP 1 737 951 B1**
(45) Date of publication and mention of the grant of the patent: **26.05.2010**
(21) Application number: 05735322.9
(22) Date of filing: 15.04.2005
(51) Int. Cl.: C12N 9/24, C12N 15/56, C12N 15/67, C12N 15/80, D21C 9/10, C12S 3/08

(54) **METHOD AND DNA CONSTRUCTS FOR INCREASING THE PRODUCTION LEVEL OF CARBOHYDRATE DEGRADING ENZYMES IN FILAMENTOUS FUNGI**
VERFAHREN UND DNA-KONSTRUKTE ZUR ERHÖHUNG DES PRODUKTIONSNIVEAUS VON KOHLENHYDRAT ABBAUENDEN ENZYMEN IN FILAMENTÖSEN PILZEN
PROCEDE ET CONSTRUCTIONS D'ADN PERMETTANT D'ACCROITRE LE NIVEAU DE PRODUCTION D'ENZYMES DEGRADANT LES GLUCIDES DANS DES CHAMPIGNONS FILAMENTEUX

(30) Priority: 16.04.2004 US 562692 P; 16.04.2004 FI 20040551
(43) Date of publication of application: 03.01.2007
(73) Proprietor: AB Enzymes Oy, 05201 Rajamäki (FI)
(72) Inventor: PALOHEIMO, Marja, FI-01450 Vantaa (FI); MÄNTYLÄ, Arja, FI-00400 Helsinki (FI); LESKINEN, Sanna, FI-10960 Hanko (FI); FAGERSTRÖM, Richard, FI-02260 Espoo (FI); KALLIO, Jarno, FI-04400 Järvenpää (FI); PURANEN, Terhi, FI-01900 Nurmijärvi (FI); LANTTO, Raija, FI-01800 Klaukkala (FI); SUOMINEN, Pirkko, Maple Grove, MN 55311 (US)
(74) Representative: Lönnqvist, Gunnel Solveig Kristina
(86) International application number: PCT/FI2005/050123
(87) International publication number: WO 2005/100557

(56) References cited:
- EP-A1- 1 344 820
- EP-A2- 1 408 108
- WO-A-97/15660
- WO-A-02/057538
- WO-A1-90/15860
- WO-A1-93/25693
- WO-A1-97/27306
- WO-A2-01/96382
- US-A- 5 712 142
- US-A- 6 083 734
- US-B1- 6 562 340
- HAAKANA HELI ET AL: "Cloning of cellulase genes from Melanocarpus albomyces and their efficient expression in Trichoderma reesei" ENZYME MICROB. TECHNOL.; ENZYME AND MICROBIAL TECHNOLOGY FEB 5 2004, vol. 34, no. 2, 5 February 2004 (2004-02-05), pages 159-167, XP002449357
- BLACK G W ET AL: "Cellulose binding domains and linker sequences potentiate the activity of hemicellulases against complex substrates." JOURNAL OF BIOTECHNOLOGY 16 SEP 1997, vol. 57, no. 1-3, 16 September 1997 (1997-09-16), pages 59-69, XP002449358 ISSN: 0168-1656
- LESKINEN S ET AL: "Thermostable xylanases, Xyn10A and Xyn11A, from the actinomycete Nonomuraea flexuosa: isolation of the genes and characterization of recombinant Xyn11A polypeptides produced in Trichoderma reesei" APPLIED MICROBIOLOGY AND BIOTECHNOLOGY, SPRINGER-VERLAG, BE, vol. 67, no. 4, 1 June 2005 (2005-06-01), pages 495-505, XP019331832 ISSN: 1432-0614
- PALOHEIMO MARJA ET AL: "Increased production of xylanase by expression of a truncated version of the xyn11A gene from Nonomuraea flexuosa in Trichoderma reesei." APPLIED AND ENVIRONMENTAL MICROBIOLOGY MAY 2007, vol. 73, no. 10, May 2007 (2007-05), pages 3215-3224, XP002449359 ISSN: 0099-2240
- PALOHEIMO M ET AL: 'High-yield production of a bacterial xylanase in the filamentous fungus Trichoderma reesei requires a carrier polypeptide with an intact domain structure.' APPLIED AND ENVIRONMENTAL MICROBIOLOGY. vol. 69, no. 12, December 2003, pages 7073 - 7082, XP002363277
- RIXON J E ET AL: 'Do the non-catalytic polysaccharide-binding domains and linker regions enhance the biobleaching properties of modular xylanases.' APPLIED MICROBIOLOGY AND BIOTECHNOLOGY. vol. 46, no. 5-6, December 1996, pages 514 - 520, XP008087437

### SEQUENCE LISTING

<110> AB Enzymas Oy
<120> METHOD AND DNA CONSTRUCTS FOR INCREASING THE PRODUCTION LEVEL OF CARBOHYDRATE DEGRADING ENZYNES IN FILAMENTOUS FUNGI
<130> A3834PC EP
<150> US 06/ 562, 692
   <151> 2004-04-16
<150> FI 20040551
   <151> 2004-04-16
<150> PCT/ Fl 2005/ 050123
   <151> 2005-04-15
<160> 25
<170> Patent Inversion 3.3
<210> 1
   <211> 1375
   <212> DNA
   <213> Nonomuraea flexuosa
<220>
   <221> Nf xyn11A nucleotide sequence (AJ508952), the coding region is from nt 303 to nt 1337
   <222> (1)..(1375)
<400> 1
   cccgggtatt catgtgaatg attagcaaca gttatgttac ggagatattt ctgagagtgt     60
   tgacaggtcg tgaagtcggt ccgatacttt cgagctagct ccgatagttt tcgatacgcc    120
   ggcacatcga gcacgtcgga cgagtcacgc gccacgtcgg ttttccgccg cacgccgcgc    180
   agagcggccg gagaaccccc gcgtgtccgc ggcatcggtg ccggtccgtc gttcgccgcc    240
   gaccgcgcgc cgggtcgcga cacgccagcc cccatcggcc cttcttcacg aggaagccgt    300
   acatgaacga acccctcacc atcacgcagg ccaggcgccg cagacgcctc ggcctccggc    360
   gcatcgtcac cagtgccttc gccctggcac tcgccatcgc cggtgcgctg ctgcccggca    420
   cggcccacgc cgacaccacc atcacccaga accagaccgg gtacgacaac ggctacttct    480
   actcgttctg gaccgacgcg cccgggaccg tctccatgac cctccactcg ggcggcagct    540
   acagcacctc gtggcggaac accgggaact tcgtcgccgg caagggctgg tccaccggcg    600
   gacggcggac cgtgacctac aacgcctcct tcaacccgtc gggtaacgcc tacctcacgc    660
   tctacggctg gaccaggaac ccgctcgtcg agtactacat cgtcgagagc tggggcacct    720
   accggcccac cggcacctac aagggcaccg tcaccaccga cggcggcacg tacgacatct    780
   acgagacctg gcggtacaac gcgccgtcca tcgagggcac ccggaccttc cagcagttct    840
   ggagcgtccg gcagcagaag cggaccagcg gcaccatcac catcggcaac cacttcgacg    900
   cctgggcccg cgccggcatg aacctgggca gccacgacta ccagatcatg gcgaccgagg    960
   gctaccagag cagcggtagc tccaccgtct ccatcagcga gggtggcaac cccggcaacc   1020
   cgggtaaccc cggcaacccc ggcaaccccg gtaacccggg taaccccggc ggtggctgcg   1080
   tcgcgaccct ctccgccggc cagcagtgga gcgaccgcta caacctcaac gtctcggtca   1140
   gcggctcgaa caactggacg gtccggatgg acgtgcccta cccggcccgc atcatcgcca   1200
   cctggaacat ccacgcccag tggcccgagt cccaggtgct catcgccaga cccaacggca   1260
   acggcaacaa ctggggcgtg acgatccagc acaacggcaa ctggacctgg ccgacggtca   1320
   cctgtaccgc gaactgagtt cccgccccca aaggtggcgc ggcggctccc ggccg        1375
<210> 2
   <211> 906
   <212> DNA
   <213> Nonomuraea flexuosa
<220>
   <221> am35, Nf xyn11A coding region for the mature Nf Xyn11A (AM35) protein
   <222> (1)..(906)
<400> 2
   gacaccacca tcacccagaa ccagaccggg tacgacaacg gctacttcta ctcgttctgg     60
   accgacgcgc ccgggaccgt ctccatgacc ctccactcgg gcggcagcta cagcacctcg    120
   tggcggaaca ccgggaactt cgtcgccggc aagggctggt ccaccggcgg acggcggacc    180
   gt gacct aca acgcct cct t caacccgt cg ggt aacgcct acct cacgct ct acggct gg 240
   accaggaacc cgctcgtcga gtactacatc gtcgagagct ggggcaccta ccggcccacc    300
   ggcacctaca agggcaccgt caccaccgac ggcggcacgt acgacatcta cgagacctgg    360
   cggtacaacg cgccgtccat cgagggcacc cggaccttcc agcagttctg gagcgtccgg    420
   cagcagaagc ggaccagcgg caccatcacc atcggcaacc acttcgacgc ctgggcccgc    480
   gccggcatga acctgggcag ccacgactac cagatcatgg cgaccgaggg ctaccagagc    540
   agcggtagct ccaccgtctc catcagcgag ggtggcaacc ccggcaaccc gggtaacccc    600
   ggcaaccccg gcaaccccgg taacccgggt aaccccggcg gtggctgcgt cgcgaccctc    660
   t ccgccggcc agcagt ggag cgaccgct ac aacct caacg t ct cggt cag cggct cgaac 720
   aactggacgg tccggatgga cgtgccctac ccggcccgca tcatcgccac ctggaacatc    780
   cacgcccagt ggcccgagtc ccaggtgctc atcgccagac ccaacggcaa cggcaacaac    840
   tggggcgtga cgatccagca caacggcaac tggacctggc cgacggtcac ctgtaccgcg    900
   aact ga 906
<210> 3
   <211> 663
   <212> DNA
   <213> Nonorruraea flexuosa
<220>
   <221> am24, shortened form of am35, includes a STOP codon
   <222> (1)..(663)
<400> 3
   gacaccacca tcacccagaa ccagaccggg tacgacaacg gctacttcta ctcgttctgg     60
   accgacgcgc ccgggaccgt ctccatgacc ctccactcgg gcggcagcta cagcacctcg    120
   tggcggaaca ccgggaactt cgtcgccggc aagggctggt ccaccggcgg acggcggacc    180
   gt gacct aca acgcct cct t caacccgt cg ggt aacgcct acct cacgct ct acggct gg 240
   accaggaacc cgctcgtcga gtactacatc gtcgagagct ggggcaccta ccggcccacc    300
   ggcacctaca agggcaccgt caccaccgac ggcggcacgt acgacatcta cgagacctgg    360
   cggtacaacg cgccgtccat cgagggcacc cggaccttcc agcagttctg gagcgtccgg    420
   cagcagaagc ggaccagcgg caccatcacc atcggcaacc acttcgacgc ctgggcccgc    480
   gccggcatga acctgggcag ccacgactac cagatcatgg cgaccgaggg ctaccagagc    540
   agcggtagct ccaccgtctc catcagcgag ggtggcaacc ccggcaaccc gggtaacccc    600
   ggcaaccccg gcaaccccgg taacccgggt aaccccggcg gtggctgcgt cgcgaccctc    660
   t aa 663
<210> 4
   <211> 906
   <212> DNA
   <213> Nonomuraea flexuosa
<220>
   <221> am35*, like am35 but 9 codons are changed in the sequence (Example 10) (the changes do not alter the encoded amino acid sequence)
   <222> (1)..(906)
<400> 4
   gacaccacca tcacccagaa ccagaccggc tacgacaacg gctacttcta ctcgttctgg      60
   accgacgccc ccggcaccgt ctccatgacc ctccactcgg gcggcagcta cagcacctcg     120
   tggcgcaaca ccggcaactt cgtcgccggc aagggctggt ccaccggcgg ccgccgcacc     180
   gt cacct aca acgcct cct t caacccgt cg ggt aacgcct acct cacgct ct acggct gg 240
   accaggaacc cgctcgtcga gtactacatc gtcgagagct ggggcaccta ccggcccacc     300
   ggcacctaca agggcaccgt caccaccgac ggcggcacgt acgacatcta cgagacctgg     360
   cggtacaacg cgccgtccat cgagggcacc cggaccttcc agcagttctg gagcgtccgg     420
   cagcagaagc ggaccagcgg caccatcacc atcggcaacc acttcgacgc ctgggcccgc     480
   gccggcatga acctgggcag ccacgactac cagatcatgg cgaccgaggg ctaccagagc     540
   agcggtagct ccaccgtctc catcagcgag ggtggcaacc ccggcaaccc gggtaacccc     600
   ggcaaccccg gcaaccccgg taacccgggt aaccccggcg gtggctgcgt cgcgaccctc     660
   t ccgccggcc agcagt ggag cgaccgct ac aacct caacg t ct cggt cag cggct cgaac 720
   aactggacgg tccggatgga cgtgccctac ccggcccgca tcatcgccac ctggaacatc     780
   cacgcccagt ggcccgagtc ccaggtgctc atcgccagac ccaacggcaa cggcaacaac     840
   tggggcgtga cgatccagca caacggcaac tggacctggc cgacggtcac ctgtaccgcg     900
   aact ga 906
<210> 5
   <211> 663
   <212> DNA
   <213> Nonomuraea flexuosa
<220>
   <221> arr24* , like arr24 but 9 codons are changed in the sequence like in am35* (Example 10)
   <222> (1)..(663)
<400> 5
   gacaccacca tcacccagaa ccagaccggc tacgacaacg gctacttcta ctcgttctgg      60
   accgacgccc ccggcaccgt ctccatgacc ctccactcgg gcggcagcta cagcacctcg     120
   tggcgcaaca ccggcaactt cgtcgccggc aagggctggt ccaccggcgg ccgccgcacc     180
   gt cacct aca acgcct cct t caacccgt cg ggt aacgcct acct cacgct ct acggct gg 240
   accaggaacc cgctcgtcga gtactacatc gtcgagagct ggggcaccta ccggcccacc     300
   ggcacctaca agggcaccgt caccaccgac ggcggcacgt acgacatcta cgagacctgg     360
   cggtacaacg cgccgtccat cgagggcacc cggaccttcc agcagttctg gagcgtccgg     420
   cagcagaagc ggaccagcgg caccatcacc atcggcaacc acttcgacgc ctgggcccgc     480
   gccggcatga acctgggcag ccacgactac cagatcatgg cgaccgaggg ctaccagagc     540
   agcggtagct ccaccgtctc catcagcgag ggtggcaacc ccggcaaccc gggtaacccc     600
   ggcaaccccg gcaaccccgg taacccgggt aaccccggcg gtggctgcgt cgcgaccctc     660
   t aa 663
<210> 6
   <211> 1864
   <212> DNA
   <213> Nonorruraea flexuosa
<220>
   <221> Nf xyn10A nucleotide sequence (AJ508953), the coding region is from nt 194 to nt 1672
   <222> (1)..(1864)
<400> 6
   ttcggcagcc tattgacaaa tttcgtgaat gtttcccaca cttgctctgc agacggcccc      60
   gccgatcatg ggtgcaccgg tcggcgggac cgtgctccga cgccattcgg gggtgtgcgc     120
   ctgcgggcgc ggcgtcgatc ccgcggggac tcccgcggtt ccctttccgt gtccctctaa     180
   tggaggctca ggcatgggcg tgaacgcctt ccccagaccc ggagctcggc ggttcaccgg     240
   cgggctgtac cgggccctgg ccgcggccac ggtgagcgtg gtcggcgtgg tcacggccct     300
   gacggtgacc cagcccgcca gcgccgcggc gagcacgctc gccgagggtg ccgcgcagca     360
   caaccggtac ttcggcgtgg ccatcgccgc gaacaggctc accgactcgg tctacaccaa     420
   catcgcgaac cgcgagttca actcggtgac ggccgagaac gagatgaaga tcgacgccac     480
   cgagccgcag caggggcggt tcgacttcac ccaggccgac cggatctaca actgggcgcg     540
   ccagaacggc aagcaggtcc gcggccacac cctggcctgg cactcgcagc agccgcagtg     600
   gat gcagaac ct cagcggcc aggcgct gcg ccaggcgat g at caaccaca t ccagggggt 660
   cat gt cct ac t accggggca agat cccgat ct gggacgt g gt gaacgagg cgt t cgagga 720
   cggaaactcc ggccgccggt gcgactccaa cctccagcgc accggtaacg attggatcga     780
   ggtcgcgttc cgcaccgccc gccaggggga cccctcggcc aagctctgct acaacgacta     840
   caacatcgag aactggaacg cggccaagac ccaggcggtc tacaacatgg tgcgggactt     900
   caagt cccgc ggcgt gccca t cgact gcgt gggct t ccag t cgcact t ca acagcggt aa 960
   cccgtacaac ccgaacttcc gcaccaccct gcagcagttc gcggccctcg gcgtggacgt    1020
   cgaggtcacc gagctggaca tcgagaacgc cccggcccag acctacgcca gcgtgatccg    1080
   ggactgcctg gccgtggacc gctgcaccgg catcaccgtc tggggtgtcc gcgacagcga    1140
   ctcctggcgc tcgtaccaga acccgctgct gttcgacaac aacggcaaca agaagcaggc    1200
   ct act acgcg gt gct cgacg ccct gaacga gggct ccgac gacggt ggcg gcccgt ccaa 1260
   cccgccggt c t cgccgccgc cgggt ggcgg t t ccgggcag at ccggggcg t ggcct ccaa 1320
   ccggtgcatc gacgtgccga acggcaacac cgccgacggc acccaggtcc agctgtacga    1380
   ct gccacagc ggt t ccaacc agcagt ggac ct acacct cg t ccggt gagt t ccgcat ct t 1440
   cggcaacaag tgcctggacg cgggcggctc cagcaacggt gcggtggtcc agatctacag    1500
   ctgctggggc ggcgccaacc agaagtggga gctccgggcc gacggcacca tcgtgggcgt    1560
   gcagtccggg ctgtgcctcg acgcggtggg tggcggcacc ggcaacggca cgcggctgca    1620
   gctctactcc tgctggggcg gcaacaacca gaagtggtcc tacaacgcct gatccccggc    1680
   tgatcgaccc tagttgaggc cgtctccggt acggcaccgt cggaccggag gcggtccctt    1740
   gttcgtccag gacggaagga ccggtctgag caggcgcggc gatcggacac catggtggga    1800
   ggcacgaaag cgggaggggg tcgtattccg agactccggg aagtggaggt gttcctccac    1860
   ct ga 1864
<210> 7
   <211> 1347
   <212> DNA
   <213> Nonomuraea flexuosa
<220>
   <221> am50, Nf xyn10A coding region for the mature Nf Xyn10A (AM50) protein
   <222> (1)..(1347)
<400> 7
   gcggcgagca cgctcgccga gggtgccgcg cagcacaacc ggtacttcgg cgtggccatc 60
   gccgcgaaca ggctcaccga ctcggtctac accaacatcg cgaaccgcga gttcaactcg 120
   gtgacggccg agaacgagat gaagatcgac gccaccgagc cgcagcaggg gcggttcgac 180
   ttcacccagg ccgaccggat ctacaactgg gcgcgccaga acggcaagca ggtccgcggc 240
   cacaccctgg cctggcactc gcagcagccg cagtggatgc agaacctcag cggccaggcg 300
   ctgcgccagg cgatgatcaa ccacatccag ggggtcatgt cctactaccg gggcaagatc 360
   ccgatctggg acgtggtgaa cgaggcgttc gaggacggaa actccggccg ccggtgcgac 420
   tccaacctcc agcgcaccgg taacgattgg atcgaggtcg cgttccgcac cgcccgccag 480
   ggggacccct cggccaagct ctgctacaac gactacaaca tcgagaactg gaacgcggcc 540
   aagacccagg cggtctacaa catggtgcgg gacttcaagt cccgcggcgt gcccatcgac 600
   tgcgtgggct tccagtcgca cttcaacagc ggtaacccgt acaacccgaa cttccgcacc 660
   accctgcagc agttcgcggc cctcggcgtg gacgtcgagg tcaccgagct ggacatcgag 720
   aacgccccgg cccagaccta cgccagcgtg atccgggact gcctggccgt ggaccgctgc 780
   accggcatca ccgtctgggg tgtccgcgac agcgactcct ggcgctcgta ccagaacccg 840
   ctgctgttcg acaacaacgg caacaagaag caggcctact acgcggtgct cgacgccctg 900
   aacgagggct ccgacgacgg tggcggcccg tccaacccgc cggtctcgcc gccgccgggt 960
   ggcggttccg ggcagatccg gggcgtggcc tccaaccggt gcatcgacgt gccgaacggc 1020
   aacaccgccg acggcaccca ggtccagctg tacgactgcc acagcggttc caaccagcag 1080
   tggacctaca cctcgtccgg tgagttccgc atcttcggca acaagtgcct ggacgcgggc 1140
   ggctccagca acggtgcggt ggtccagatc tacagctgct ggggcggcgc caaccagaag 1200
   tgggagctcc gggccgacgg caccatcgtg ggcgtgcagt ccgggctgtg cctcgacgcg 1260
   gtgggtggcg gcaccggcaa cggcacgcgg ctgcagctct actcctgctg gggcggcaac 1320
   aaccagaagt ggtcctacaa cgcctaa 1347
<210> 8
   <211> 972
   <212> DNA
   <213> Nonomuraea flexuosa
<220>
   <221> The sequence encoding the AM50 core and linker regions, includes a STOP codon
   <222> (1)..(972)
<400> 8
   gcggcgagca cgctcgccga gggtgccgcg cagcacaacc ggtacttcgg cgtggccatc 60
   gccgcgaaca ggctcaccga ctcggtctac accaacatcg cgaaccgcga gttcaactcg 120
   gtgacggccg agaacgagat gaagatcgac gccaccgagc cgcagcaggg gcggttcgac 180
   ttcacccagg ccgaccggat ctacaactgg gcgcgccaga acggcaagca ggtccgcggc 240
   cacaccctgg cctggcactc gcagcagccg cagtggatgc agaacctcag cggccaggcg 300
   ctgcgccagg cgatgatcaa ccacatccag ggggtcatgt cctactaccg gggcaagatc 360
   ccgatctggg acgtggtgaa cgaggcgttc gaggacggaa actccggccg ccggtgcgac 420
   tccaacctcc agcgcaccgg taacgattgg atcgaggtcg cgttccgcac cgcccgccag 480
   ggggacccct cggccaagct ctgctacaac gactacaaca tcgagaactg gaacgcggcc 540
   aagacccagg cggtctacaa catggtgcgg gacttcaagt cccgcggcgt gcccatcgac 600
   tgcgtgggct tccagtcgca cttcaacagc ggtaacccgt acaacccgaa cttccgcacc 660
   accctgcagc agttcgcggc cctcggcgtg gacgtcgagg tcaccgagct ggacatcgag 720
   aacgccccgg cccagaccta cgccagcgtg atccgggact gcctggccgt ggaccgctgc 780
   accggcatca ccgtctgggg tgtccgcgac agcgactcct ggcgctcgta ccagaacccg 840
   ctgctgttcg acaacaacgg caacaagaag caggcctact acgcggtgct cgacgccctg 900
   aacgagggct ccgacgacgg tggcggcccg tccaacccgc cggtctcgcc gccgccgggt 960
   ggcggttcct aa 972
<210> 9
   <211> 906
   <212> DNA
   <213> Nonomuraea flexuosa
<220>
   <221> The sequence encoding the AM50 core region, includes a STCP codon
   <222> (1)..(906)
<400> 9
   gcggcgagca cgctcgccga gggtgccgcg cagcacaacc ggtacttcgg cgtggccatc 60
   gccgcgaaca ggctcaccga ctcggtctac accaacatcg cgaaccgcga gttcaactcg 120
   gtgacggccg agaacgagat gaagatcgac gccaccgagc cgcagcaggg gcggttcgac 180
   ttcacccagg ccgaccggat ctacaactgg gcgcgccaga acggcaagca ggtccgcggc 240
   cacaccctgg cctggcactc gcagcagccg cagtggatgc agaacctcag cggccaggcg 300
   ctgcgccagg cgatgatcaa ccacatccag ggggtcatgt cctactaccg gggcaagatc 360
   ccgatctggg acgtggtgaa cgaggcgttc gaggacggaa actccggccg ccggtgcgac 420
   tccaacctcc agcgcaccgg taacgattgg atcgaggtcg cgttccgcac cgcccgccag 480
   ggggacccct cggccaagct ctgctacaac gactacaaca tcgagaactg gaacgcggcc 540
   aagacccagg cggtctacaa catggtgcgg gacttcaagt cccgcggcgt gcccatcgac 600
   tgcgtgggct tccagtcgca cttcaacagc ggtaacccgt acaacccgaa cttccgcacc 660
   accctgcagc agttcgcggc cctcggcgtg gacgtcgagg tcaccgagct ggacatcgag 720
   aacgccccgg cccagaccta cgccagcgtg atccgggact gcctggccgt ggaccgctgc 780
   accggcatca ccgtctgggg tgtccgcgac agcgactcct ggcgctcgta ccagaacccg 840
   ctgctgttcg acaacaacgg caacaagaag caggcctact acgcggtgct cgacgccctg 900
   aactaa 906
<210> 10
   <211> 344
   <212> PRT
   <213> Nonomuraea flexuosa
<220>
   <221> Nf Xyn11A amino acid sequence (AJ508952) encoded by the Nf xyn11A gene
   <222> (1)..(344)
<400> 10
   Met Asn Glu Pro Leu Thr Ile Thr Gln Ala Arg Arg Arg Arg Arg Leu
   1 5 10 15
   Gly Leu Arg Arg Ile Val Thr Ser Ala Phe Ala Leu Ala Leu Ala Ile
   20 25 30
   Ala Gly Ala Leu Leu Pro Gly Thr Ala His Ala Asp Thr Thr Ile Thr
   35 40 45
   Gln Asn Gln Thr Gly Tyr Asp Asn Gly Tyr Phe Tyr Ser Phe Trp Thr
   50 55 60
   Asp Ala Pro Gly Thr Val Ser Met Thr Leu His Ser Gly Gly Ser Tyr
   65 70 75 80
   Ser Thr Ser Trp Arg Asn Thr Gly Asn Phe Val Ala Gly Lys Gly Trp
   85 90 95
   Ser Thr Gly Gly Arg Arg Thr Val Thr Tyr Asn Ala Ser Phe Asn Pro
   100 105 110
   Ser Gly Asn Ala Tyr Leu Thr Leu Tyr Gly Trp Thr Arg Asn Pro Leu
   115 120 125
   Val Glu Tyr Tyr Ile Val Glu Ser Trp Gly Thr Tyr Arg Pro Thr Gly
   130 135 140
   Thr Tyr Lys Gly Thr Val Thr Thr Asp Gly Gly Thr Tyr Asp Ile Tyr
   145 150 155 160
   Glu Thr Trp Arg Tyr Asn Ala Pro Ser Ile Glu Gly Thr Arg Thr Phe
   165 170 175
   Gln Gln Phe Trp Ser Val Arg Gln Gln Lys Arg Thr Ser Gly Thr Ile
   180 185 190
   Thr Ile Gly Asn His Phe Asp Ala Trp Ala Arg Ala Gly Met Asn Leu
   195 200 205
   Gly Ser His Asp Tyr Gln Ile Met Ala Thr Glu Gly Tyr Gln Ser Ser
   210 215 220
   Gly Ser Ser Thr Val Ser Ile Ser Glu Gly Gly Asn Pro Gly Asn Pro
   225 230 235 240
   Gly Asn Pro Gly Asn Pro Gly Asn Pro Gly Asn Pro Gly Asn Pro Gly
   245 250 255
   Gly Gly Cys Val Ala Thr Leu Ser Ala Gly Gln Gln Trp Ser Asp Arg
   260 265 270
   Tyr Asn Leu Asn Val Ser Val Ser Gly Ser Asn Asn Trp Thr Val Arg
   275 280 285
   Met Asp Val Pro Tyr Pro Ala Arg Ile Ile Ala Thr Trp Asn Ile His
   290 295 300
   Ala Gln Trp Pro Glu Ser Gln Val Leu Ile Ala Arg Pro Asn Gly Asn
   305 310 315 320
   Gly Asn Asn Trp Gly Val Thr Ile Gln His Asn Gly Asn Trp Thr Trp
   325 330 335
   Pro Thr Val Thr Cys Thr Ala Asn
   340
<210> 11
   <211> 301
   <212> PRT
   <213> Nonomuraea flexuosa
<220>
   <221> r33. 4 kDa = AM35 = full length mature Nf Xyn11A protein, encoded by am35 and am35* genes
   <222> (1)..(301)
<400> 11
   Asp Thr Thr Ile Thr Gln Asn Gln Thr Gly Tyr Asp Asn Gly Tyr Phe
   1 5 10 15
   Tyr Ser Phe Trp Thr Asp Ala Pro Gly Thr Val Ser Met Thr Leu His
   20 25 30
   Ser Gly Gly Ser Tyr Ser Thr Ser Trp Arg Asn Thr Gly Asn Phe Val
   35 40 45
   Ala Gly Lys Gly Trp Ser Thr Gly Gly Arg Arg Thr Val Thr Tyr Asn
   50 55 60
   Ala Ser Phe Asn Pro Ser Gly Asn Ala Tyr Leu Thr Leu Tyr Gly Trp
   65 70 75 80
   Thr Arg Asn Pro Leu Val Glu Tyr Tyr Ile Val Glu Ser Trp Gly Thr
   85 90 95
   Tyr Arg Pro Thr Gly Thr Tyr Lys Gly Thr Val Thr Thr Asp Gly Gly
   100 105 110
   Thr Tyr Asp Ile Tyr Glu Thr Trp Arg Tyr Asn Ala Pro Ser Ile Glu
   115 120 125
   Gly Thr Arg Thr Phe Gln Gln Phe Trp Ser Val Arg Gln Gln Lys Arg
   130 135 140
   Thr Ser Gly Thr Ile Thr Ile Gly Asn His Phe Asp Ala Trp Ala Arg
   145 150 155 160
   Ala Gly Met Asn Leu Gly Ser His Asp Tyr Gln Ile Met Ala Thr Glu
   165 170 175
   Gly Tyr Gln Ser Ser Gly Ser Ser Thr Val Ser Ile Ser Glu Gly Gly
   180 185 190
   Asn Pro Gly Asn Pro Gly Asn Pro Gly Asn Pro Gly Asn Pro Gly Asn
   195 200 205
   Pro Gly Asn Pro Gly Gly Gly Cys Val Ala Thr Leu Ser Ala Gly Gln
   210 215 220
   Gln Trp Ser Asp Arg Tyr Asn Leu Asn Val Ser Val Ser Gly Ser Asn
   225 230 235 240
   Asn Trp Thr Val Arg Met Asp Val Pro Tyr Pro Ala Arg Ile Ile Ala
   245 250 255
   Thr Trp Asn Ile His Ala Gln Trp Pro Glu Ser Gln Val Leu Ile Ala
   260 265 270
   Arg Pro Asn Gly Asn Gly Asn Asn Trp Gly Val Thr Ile Gln His Asn
   275 280 285
   Gly Asn Trp Thr Trp Pro Thr Val Thr Cys Thr Ala Asn
   290 295 300
<210> 12
   <211> 220
   <212> PRT
   <213> Nonomuraea flexuosa
<220>
   <221> AM24, truncated form from AM35, encoded by am24 and am24* genes
   <222> (1)..(220)
<400> 12
   Asp Thr Thr Ile Thr Gln Asn Gln Thr Gly Tyr Asp Asn Gly Tyr Phe
   1 5 10 15
   Tyr Ser Phe Trp Thr Asp Ala Pro Gly Thr Val Ser Met Thr Leu His
   20 25 30
   Ser Gly Gly Ser Tyr Ser Thr Ser Trp Arg Asn Thr Gly Asn Phe Val
   35 40 45
   Ala Gly Lys Gly Trp Ser Thr Gly Gly Arg Arg Thr Val Thr Tyr Asn
   50 55 60
   Ala Ser Phe Asn Pro Ser Gly Asn Ala Tyr Leu Thr Leu Tyr Gly Trp
   65 70 75 80
   Thr Arg Asn Pro Leu Val Glu Tyr Tyr Ile Val Glu Ser Trp Gly Thr
   85 90 95
   Tyr Arg Pro Thr Gly Thr Tyr Lys Gly Thr Val Thr Thr Asp Gly Gly
   100 105 110
   Thr Tyr Asp Ile Tyr Glu Thr Trp Arg Tyr Asn Ala Pro Ser Ile Glu
   115 120 125
   Gly Thr Arg Thr Phe Gln Gln Phe Trp Ser Val Arg Gln Gln Lys Arg
   130 135 140
   Thr Ser Gly Thr Ile Thr Ile Gly Asn His Phe Asp Ala Trp Ala Arg
   145 150 155 160
   Ala Gly Met Asn Leu Gly Ser His Asp Tyr Gln Ile Met Ala Thr Glu
   165 170 175
   Gly Tyr Gln Ser Ser Gly Ser Ser Thr Val Ser Ile Ser Glu Gly Gly
   180 185 190
   Asn Pro Gly Asn Pro Gly Asn Pro Gly Asn Pro Gly Asn Pro Gly Asn
   195 200 205
   Pro Gly Asn Pro Gly Gly Gly Cys Val Ala Thr Leu
   210 215 220
<210> 13
   <211> 217
   <212> PRT
   <213> Nonomuraea flexuosa
<220>
   <221> r 23. 8 kDa, t r uncat ed form from AM35
   <222> (1)..(217)
<400> 13
   Asp Thr Thr Ile Thr Gln Asn Gln Thr Gly Tyr Asp Asn Gly Tyr Phe
   1 5 10 15
   Tyr Ser Phe Trp Thr Asp Ala Pro Gly Thr Val Ser Met Thr Leu His
   20 25 30
   Ser Gly Gly Ser Tyr Ser Thr Ser Trp Arg Asn Thr Gly Asn Phe Val
   35 40 45
   Ala Gly Lys Gly Trp Ser Thr Gly Gly Arg Arg Thr Val Thr Tyr Asn
   50 55 60
   Ala Ser Phe Asn Pro Ser Gly Asn Ala Tyr Leu Thr Leu Tyr Gly Trp
   65 70 75 80
   Thr Arg Asn Pro Leu Val Glu Tyr Tyr Ile Val Glu Ser Trp Gly Thr
   85 90 95
   Tyr Arg Pro Thr Gly Thr Tyr Lys Gly Thr Val Thr Thr Asp Gly Gly
   100 105 110
   Thr Tyr Asp Ile Tyr Glu Thr Trp Arg Tyr Asn Ala Pro Ser Ile Glu
   115 120 125
   Gly Thr Arg Thr Phe Gln Gln Phe Trp Ser Val Arg Gln Gln Lys Arg
   130 135 140
   Thr Ser Gly Thr Ile Thr Ile Gly Asn His Phe Asp Ala Trp Ala Arg
   145 150 155 160
   Ala Gly Met Asn Leu Gly Ser His Asp Tyr Gln Ile Met Ala Thr Glu
   165 170 175
   Gly Tyr Gln Ser Ser Gly Ser Ser Thr Val Ser Ile Ser Glu Gly Gly
   180 185 190
   Asn Pro Gly Asn Pro Gly Asn Pro Gly Asn Pro Gly Asn Pro Gly Asn
   195 200 205
   Pro Gly Asn Pro Gly Gly Gly Cys Val
   210 215
<210> 14
   <211> 193
   <212> PRT
   <213> Nonomuraea flexuosa
<220>
   <221> r 22. 0 kDa, truncated form from AM35
   <222> (1)..(193)
<400> 14
   Asp Thr Thr Ile Thr Gln Asn Gln Thr Gly Tyr Asp Asn Gly Tyr Phe
   1 5 10 15
   Tyr Ser Phe Trp Thr Asp Ala Pro Gly Thr Val Ser Met Thr Leu His
   20 25 30
   Ser Gly Gly Ser Tyr Ser Thr Ser Trp Arg Asn Thr Gly Asn Phe Val
   35 40 45
   Ala Gly Lys Gly Trp Ser Thr Gly Gly Arg Arg Thr Val Thr Tyr Asn
   50 55 60
   Ala Ser Phe Asn Pro Ser Gly Asn Ala Tyr Leu Thr Leu Tyr Gly Trp
   65 70 75 80
   Thr Arg Asn Pro Leu Val Glu Tyr Tyr Ile Val Glu Ser Trp Gly Thr
   85 90 95
   Tyr Arg Pro Thr Gly Thr Tyr Lys Gly Thr Val Thr Thr Asp Gly Gly
   100 105 110
   Thr Tyr Asp Ile Tyr Glu Thr Trp Arg Tyr Asn Ala Pro Ser Ile Glu
   115 120 125
   Gly Thr Arg Thr Phe Gln Gln Phe Trp Ser Val Arg Gln Gln Lys Arg
   130 135 140
   Thr Ser Gly Thr Ile Thr Ile Gly Asn His Phe Asp Ala Trp Ala Arg
   145 150 155 160
   Ala Gly Met Asn Leu Gly Ser His Asp Tyr Gln Ile Met Ala Thr Glu
   165 170 175
   Gly Tyr Gln Ser Ser Gly Ser Ser Thr Val Ser Ile Ser Glu Gly Gly
   180 185 190
   Asn
<210> 15
   <211> 492
   <212> PRT
   <213> Nonomuraea flexuosa
<220>
   <221> Nf Xyn10A, the amino acid sequence (AJ508953) encoded by the Nf xyn10A gene
   <222> (1)..(492)
<400> 15
   Met Gly Val Asn Ala Phe Pro Arg Pro Gly Ala Arg Arg Phe Thr Gly
   1 5 10 15
   Gly Leu Tyr Arg Ala Leu Ala Ala Ala Thr Val Ser Val Val Gly Val
   20 25 30
   Val Thr Ala Leu Thr Val Thr Gln Pro Ala Ser Ala Ala Ala Ser Thr
   35 40 45
   Leu Ala Glu Gly Ala Ala Gln His Asn Arg Tyr Phe Gly Val Ala Ile
   50 55 60
   Ala Ala Asn Arg Leu Thr Asp Ser Val Tyr Thr Asn Ile Ala Asn Arg
   65 70 75 80
   Glu Phe Asn Ser Val Thr Ala Glu Asn Glu Met Lys Ile Asp Ala Thr
   85 90 95
   Glu Pro Gln Gln Gly Arg Phe Asp Phe Thr Gln Ala Asp Arg Ile Tyr
   100 105 110
   Asn Trp Ala Arg Gln Asn Gly Lys Gln Val Arg Gly His Thr Leu Ala
   115 120 125
   Trp His Ser Gln Gln Pro Gln Trp Met Gln Asn Leu Ser Gly Gln Ala
   130 135 140
   Leu Arg Gln Ala Met Ile Asn His Ile Gln Gly Val Met Ser Tyr Tyr
   145 150 155 160
   Arg Gly Lys Ile Pro Ile Trp Asp Val Val Asn Glu Ala Phe Glu Asp
   165 170 175
   Gly Asn Ser Gly Arg Arg Cys Asp Ser Asn Leu Gln Arg Thr Gly Asn
   180 185 190
   Asp Trp Ile Glu Val Ala Phe Arg Thr Ala Arg Gln Gly Asp Pro Ser
   195 200 205
   Ala Lys Leu Cys Tyr Asn Asp Tyr Asn Ile Glu Asn Trp Asn Ala Ala
   210 215 220
   Lys Thr Gln Ala Val Tyr Asn Met Val Arg Asp Phe Lys Ser Arg Gly
   225 230 235 240
   Val Pro Ile Asp Cys Val Gly Phe Gln Ser His Phe Asn Ser Gly Asn
   245 250 255
   Pro Tyr Asn Pro Asn Phe Arg Thr Thr Leu Gln Gln Phe Ala Ala Leu
   260 265 270
   Gly Val Asp Val Glu Val Thr Glu Leu Asp Ile Glu Asn Ala Pro Ala
   275 280 285
   Gln Thr Tyr Ala Ser Val Ile Arg Asp Cys Leu Ala Val Asp Arg Cys
   290 295 300
   Thr Gly Ile Thr Val Trp Gly Val Arg Asp Ser Asp Ser Trp Arg Ser
   305 310 315 320
   Tyr Gln Asn Pro Leu Leu Phe Asp Asn Asn Gly Asn Lys Lys Gln Ala
   325 330 335
   Tyr Tyr Ala Val Leu Asp Ala Leu Asn Glu Gly Ser Asp Asp Gly Gly
   340 345 350
   Gly Pro Ser Asn Pro Pro Val Ser Pro Pro Pro Gly Gly Gly Ser Gly
   355 360 365
   Gln Ile Arg Gly Val Ala Ser Asn Arg Cys Ile Asp Val Pro Asn Gly
   370 375 380
   Asn Thr Ala Asp Gly Thr Gln Val Gln Leu Tyr Asp Cys His Ser Gly
   385 390 395 400
   Ser Asn Gln Gln Trp Thr Tyr Thr Ser Ser Gly Glu Phe Arg Ile Phe
   405 410 415
   Gly Asn Lys Cys Leu Asp Ala Gly Gly Ser Ser Asn Gly Ala Val Val
   420 425 430
   Gln Ile Tyr Ser Cys Trp Gly Gly Ala Asn Gln Lys Trp Glu Leu Arg
   435 440 445
   Ala Asp Gly Thr Ile Val Gly Val Gln Ser Gly Leu Cys Leu Asp Ala
   450 455 460
   Val Gly Gly Gly Thr Gly Asn Gly Thr Arg Leu Gln Leu Tyr Ser Cys
   465 470 475 480
   Trp Gly Gly Asn Asn Gln Lys Trp Ser Tyr Asn Ala
   485 490
<210> 16
   <211> 448
   <212> PRT
   <213> Nonomuraea flexuosa
<220>
   <221> AM50, full I length mature Nf Xyn10A
   <222> (1)..(448)
<400> 16
   Ala Ala Ser Thr Leu Ala Glu Gly Ala Ala Gln His Asn Arg Tyr Phe
   1               5                   10                  15
   Gly Val Ala Ile Ala Ala Asn Arg Leu Thr Asp Ser Val Tyr Thr Asn
               20                  25                  30
   Ile Ala Asn Arg Glu Phe Asn Ser Val Thr Ala Glu Asn Glu Met Lys
           35                  40                  45
   Ile Asp Ala Thr Glu Pro Gln Gln Gly Arg Phe Asp Phe Thr Gln Ala
       50                  55                  60
   Asp Arg Ile Tyr Asn Trp Ala Arg Gln Asn Gly Lys Gln Val Arg Gly
   65                  70                  75                  80
   His Thr Leu Ala Trp His Ser Gln Gln Pro Gln Trp Met Gln Asn Leu
                   85                  90                  95
   Ser Gly Gln Ala Leu Arg Gln Ala Met Ile Asn His Ile Gln Gly Val
   100 105 110
   Met Ser Tyr Tyr Arg Gly Lys Ile Pro Ile Trp Asp Val Val Asn Glu
   115 120 125
   Ala Phe Glu Asp Gly Asn Ser Gly Arg Arg Cys Asp Ser Asn Leu Gln
   130 135 140
   Arg Thr Gly Asn Asp Trp Ile Glu Val Ala Phe Arg Thr Ala Arg Gln
   145 150 155 160
   Gly Asp Pro Ser Ala Lys Leu Cys Tyr Asn Asp Tyr Asn Ile Glu Asn
   165 170 175
   Trp Asn Ala Ala Lys Thr Gln Ala Val Tyr Asn Met Val Arg Asp Phe
   180 185 190
   Lys Ser Arg Gly Val Pro Ile Asp Cys Val Gly Phe Gln Ser His Phe
   195 200 205
   Asn Ser Gly Asn Pro Tyr Asn Pro Asn Phe Arg Thr Thr Leu Gln Gln
   210 215 220
   Phe Ala Ala Leu Gly Val Asp Val Glu Val Thr Glu Leu Asp Ile Glu
   225 230 235 240
   Asn Ala Pro Ala Gln Thr Tyr Ala Ser Val Ile Arg Asp Cys Leu Ala
   245 250 255
   Val Asp Arg Cys Thr Gly Ile Thr Val Trp Gly Val Arg Asp Ser Asp
   260 265 270
   Ser Trp Arg Ser Tyr Gln Asn Pro Leu Leu Phe Asp Asn Asn Gly Asn
   275 280 285
   Lys Lys Gln Ala Tyr Tyr Ala Val Leu Asp Ala Leu Asn Glu Gly Ser
   290 295 300
   Asp Asp Gly Gly Gly Pro Ser Asn Pro Pro Val Ser Pro Pro Pro Gly
   305 310 315 320
   Gly Gly Ser Gly Gln Ile Arg Gly Val Ala Ser Asn Arg Cys Ile Asp
   325 330 335
   Val Pro Asn Gly Asn Thr Ala Asp Gly Thr Gln Val Gln Leu Tyr Asp
   340 345 350
   Cys His Ser Gly Ser Asn Gln Gln Trp Thr Tyr Thr Ser Ser Gly Glu
   355 360 365
   Phe Arg Ile Phe Gly Asn Lys Cys Leu Asp Ala Gly Gly Ser Ser Asn
   370 375 380
   Gly Ala Val Val Gln Ile Tyr Ser Cys Trp Gly Gly Ala Asn Gln Lys
   385 390 395 400
   Trp Glu Leu Arg Ala Asp Gly Thr Ile Val Gly Val Gln Ser Gly Leu
   405 410 415
   Cys Leu Asp Ala Val Gly Gly Gly Thr Gly Asn Gly Thr Arg Leu Gln
   420 425 430
   Leu Tyr Ser Cys Trp Gly Gly Asn Asn Gln Lys Trp Ser Tyr Asn Ala
   435 440 445
<210> 17
   <211> 323
   <212> PRT
   <213> Nononruraea flexuosa
<220>
   <221> AM50 core + linker, truncated form from AM50
   <222> (1)..(323)
<400> 17
   Ala Ala Ser Thr Leu Ala Glu Gly Ala Ala Gln His Asn Arg Tyr Phe
   1 5 10 15
   Gly Val Ala Ile Ala Ala Asn Arg Leu Thr Asp Ser Val Tyr Thr Asn
   20 25 30
   Ile Ala Asn Arg Glu Phe Asn Ser Val Thr Ala Glu Asn Glu Met Lys
   35 40 45
   Ile Asp Ala Thr Glu Pro Gln Gln Gly Arg Phe Asp Phe Thr Gln Ala
   50 55 60
   Asp Arg Ile Tyr Asn Trp Ala Arg Gln Asn Gly Lys Gln Val Arg Gly
   65 70 75 80
   His Thr Leu Ala Trp His Ser Gln Gln Pro Gln Trp Met Gln Asn Leu
   85 90 95
   Ser Gly Gln Ala Leu Arg Gln Ala Met Ile Asn His Ile Gln Gly Val
   100 105 110
   Met Ser Tyr Tyr Arg Gly Lys Ile Pro Ile Trp Asp Val Val Asn Glu
   115 120 125
   Ala Phe Glu Asp Gly Asn Ser Gly Arg Arg Cys Asp Ser Asn Leu Gln
   130 135 140
   Arg Thr Gly Asn Asp Trp Ile Glu Val Ala Phe Arg Thr Ala Arg Gln
   145 150 155 160
   Gly Asp Pro Ser Ala Lys Leu Cys Tyr Asn Asp Tyr Asn Ile Glu Asn
   165 170 175
   Trp Asn Ala Ala Lys Thr Gln Ala Val Tyr Asn Met Val Arg Asp Phe
   180 185 190
   Lys Ser Arg Gly Val Pro Ile Asp Cys Val Gly Phe Gln Ser His Phe
   195 200 205
   Asn Ser Gly Asn Pro Tyr Asn Pro Asn Phe Arg Thr Thr Leu Gln Gln
   210 215 220
   Phe Ala Ala Leu Gly Val Asp Val Glu Val Thr Glu Leu Asp Ile Glu
   225 230 235 240
   Asn Ala Pro Ala Gln Thr Tyr Ala Ser Val Ile Arg Asp Cys Leu Ala
   245 250 255
   Val Asp Arg Cys Thr Gly Ile Thr Val Trp Gly Val Arg Asp Ser Asp
   260 265 270
   Ser Trp Arg Ser Tyr Gln Asn Pro Leu Leu Phe Asp Asn Asn Gly Asn
   275 280 285
   Lys Lys Gln Ala Tyr Tyr Ala Val Leu Asp Ala Leu Asn Glu Gly Ser
   290 295 300
   Asp Asp Gly Gly Gly Pro Ser Asn Pro Pro Val Ser Pro Pro Pro Gly
   305 310 315 320
   Gly Gly Ser
<210> 18
   <211> 301
   <212> PRT
   <213> Nonomuraea flexuosa
<220>
   <221> AM50 core, truncated form from AN50
   <222> (1)..(301)
<400> 18
   Ala Ala Ser Thr Leu Ala Glu Gly Ala Ala Gln His Asn Arg Tyr Phe
   1               5                   10                  15
   Gly Val Ala Ile Ala Ala Asn Arg Leu Thr Asp Ser Val Tyr Thr Asn
               20                  25                  30
   Ile Ala Asn Arg Glu Phe Asn Ser Val Thr Ala Glu Asn Glu Met Lys
           35                  40                  45
   Ile Asp Ala Thr Glu Pro Gln Gln Gly Arg Phe Asp Phe Thr Gln Ala
       50                  55                  60
   Asp Arg Ile Tyr Asn Trp Ala Arg Gln Asn Gly Lys Gln Val Arg Gly
   65                  70                  75                  80
   His Thr Leu Ala Trp His Ser Gln Gln Pro Gln Trp Met Gln Asn Leu
                   85                  90                  95
   Ser Gly Gln Ala Leu Arg Gln Ala Met Ile Asn His Ile Gln Gly Val
               100                 105                 110
   Met Ser Tyr Tyr Arg Gly Lys Ile Pro Ile Trp Asp Val Val Asn Glu
           115                 120                 125
   Ala Phe Glu Asp Gly Asn Ser Gly Arg Arg Cys Asp Ser Asn Leu Gln
       130                 135                 140
   Arg Thr Gly Asn Asp Trp Ile Glu Val Ala Phe Arg Thr Ala Arg Gln
   145                 150                 155                 160
   Gly Asp Pro Ser Ala Lys Leu Cys Tyr Asn Asp Tyr Asn Ile Glu Asn
                   165                 170                 175
   Trp Asn Ala Ala Lys Thr Gln Ala Val Tyr Asn Met Val Arg Asp Phe
               180                 185                 190
   Lys Ser Arg Gly Val Pro Ile Asp Cys Val Gly Phe Gln Ser His Phe
           195                 200                 205
   Asn Ser Gly Asn Pro Tyr Asn Pro Asn Phe Arg Thr Thr Leu Gln Gln
       210                 215                 220
   Phe Ala Ala Leu Gly Val Asp Val Glu Val Thr Glu Leu Asp Ile Glu
   225                 230                 235                 240
   Asn Ala Pro Ala Gln Thr Tyr Ala Ser Val Ile Arg Asp Cys Leu Ala
                   245                 250                 255
   Val Asp Arg Cys Thr Gly Ile Thr Val Trp Gly Val Arg Asp Ser Asp
               260                 265                 270
   Ser Trp Arg Ser Tyr Gln Asn Pro Leu Leu Phe Asp Asn Asn Gly Asn
           275                 280                 285
   Lys Lys Gln Ala Tyr Tyr Ala Val Leu Asp Ala Leu Asn
       290                 295                 300
<210> 19
   <211> 406
   <212> PRT
   <213> Nonomuraea flexuosa
<220>
   <221> AM50 core + linker + alpha/beta domains of the tail
   <222> (1)..(406)
<400> 19
   Ala Ala Ser Thr Leu Ala Glu Gly Ala Ala Gln His Asn Arg Tyr Phe
   1               5                   10                  15
   Gly Val Ala Ile Ala Ala Asn Arg Leu Thr Asp Ser Val Tyr Thr Asn
               20                  25                  30
   Ile Ala Asn Arg Glu Phe Asn Ser Val Thr Ala Glu Asn Glu Met Lys
           35                  40                  45
   Ile Asp Ala Thr Glu Pro Gln Gln Gly Arg Phe Asp Phe Thr Gln Ala
       50                  55                  60
   Asp Arg Ile Tyr Asn Trp Ala Arg Gln Asn Gly Lys Gln Val Arg Gly
   65                  70                  75                  80
   His Thr Leu Ala Trp His Ser Gln Gln Pro Gln Trp Met Gln Asn Leu
                   85                  90                  95
   Ser Gly Gln Ala Leu Arg Gln Ala Met Ile Asn His Ile Gln Gly Val
               100                 105                 110
   Met Ser Tyr Tyr Arg Gly Lys Ile Pro Ile Trp Asp Val Val Asn Glu
           115                 120                 125
   Ala Phe Glu Asp Gly Asn Ser Gly Arg Arg Cys Asp Ser Asn Leu Gln
       130                 135                 140
   Arg Thr Gly Asn Asp Trp Ile Glu Val Ala Phe Arg Thr Ala Arg Gln
   145                 150                 155                 160
   Gly Asp Pro Ser Ala Lys Leu Cys Tyr Asn Asp Tyr Asn Ile Glu Asn
                   165                 170                 175
   Trp Asn Ala Ala Lys Thr Gln Ala Val Tyr Asn Met Val Arg Asp Phe
               180                 185                 190
   Lys Ser Arg Gly Val Pro Ile Asp Cys Val Gly Phe Gln Ser His Phe
           195                 200                 205
   Asn Ser Gly Asn Pro Tyr Asn Pro Asn Phe Arg Thr Thr Leu Gln Gln
       210                 215                 220
   Phe Ala Ala Leu Gly Val Asp Val Glu Val Thr Glu Leu Asp Ile Glu
   225                 230                 235                 240
   Asn Ala Pro Ala Gln Thr Tyr Ala Ser Val Ile Arg Asp Cys Leu Ala
   245 250 255
   Val Asp Arg Cys Thr Gly Ile Thr Val Trp Gly Val Arg Asp Ser Asp
   260 265 270
   Ser Trp Arg Ser Tyr Gln Asn Pro Leu Leu Phe Asp Asn Asn Gly Asn
   275 280 285
   Lys Lys Gln Ala Tyr Tyr Ala Val Leu Asp Ala Leu Asn Glu Gly Ser
   290 295 300
   Asp Asp Gly Gly Gly Pro Ser Asn Pro Pro Val Ser Pro Pro Pro Gly
   305 310 315 320
   Gly Gly Ser Gly Gln Ile Arg Gly Val Ala Ser Asn Arg Cys Ile Asp
   325 330 335
   Val Pro Asn Gly Asn Thr Ala Asp Gly Thr Gln Val Gln Leu Tyr Asp
   340 345 350
   Cys His Ser Gly Ser Asn Gln Gln Trp Thr Tyr Thr Ser Ser Gly Glu
   355 360 365
   Phe Arg Ile Phe Gly Asn Lys Cys Leu Asp Ala Gly Gly Ser Ser Asn
   370 375 380
   Gly Ala Val Val Gln Ile Tyr Ser Cys Trp Gly Gly Ala Asn Gln Lys
   385 390 395 400
   Trp Glu Leu Arg Ala Asp
   405
<210> 20
   <211> 366
   <212> PRT
   <213> Nonomuraea flexuosa
<220>
   <221> AM50 core + linker + alpha domain of the tail
   <222> (1)..(366)
<400> 20
   Ala Ala Ser Thr Leu Ala Glu Gly Ala Ala Gln His Asn Arg Tyr Phe
   1 5 10 15
   Gly Val Ala Ile Ala Ala Asn Arg Leu Thr Asp Ser Val Tyr Thr Asn
   20 25 30
   Ile Ala Asn Arg Glu Phe Asn Ser Val Thr Ala Glu Asn Glu Met Lys
   35 40 45
   Ile Asp Ala Thr Glu Pro Gln Gln Gly Arg Phe Asp Phe Thr Gln Ala
   50 55 60
   Asp Arg Ile Tyr Asn Trp Ala Arg Gln Asn Gly Lys Gln Val Arg Gly
   65 70 75 80
   His Thr Leu Ala Trp His Ser Gln Gln Pro Gln Trp Met Gln Asn Leu
   85 90 95
   Ser Gly Gln Ala Leu Arg Gln Ala Met Ile Asn His Ile Gln Gly Val
   100 105 110
   Met Ser Tyr Tyr Arg Gly Lys Ile Pro Ile Trp Asp Val Val Asn Glu
   115 120 125
   Ala Phe Glu Asp Gly Asn Ser Gly Arg Arg Cys Asp Ser Asn Leu Gln
   130 135 140
   Arg Thr Gly Asn Asp Trp Ile Glu Val Ala Phe Arg Thr Ala Arg Gln
   145 150 155 160
   Gly Asp Pro Ser Ala Lys Leu Cys Tyr Asn Asp Tyr Asn Ile Glu Asn
   165 170 175
   Trp Asn Ala Ala Lys Thr Gln Ala Val Tyr Asn Met Val Arg Asp Phe
   180 185 190
   Lys Ser Arg Gly Val Pro Ile Asp Cys Val Gly Phe Gln Ser His Phe
   195 200 205
   Asn Ser Gly Asn Pro Tyr Asn Pro Asn Phe Arg Thr Thr Leu Gln Gln
   210 215 220
   Phe Ala Ala Leu Gly Val Asp Val Glu Val Thr Glu Leu Asp Ile Glu
   225 230 235 240
   Asn Ala Pro Ala Gln Thr Tyr Ala Ser Val Ile Arg Asp Cys Leu Ala
   245 250 255
   Val Asp Arg Cys Thr Gly Ile Thr Val Trp Gly Val Arg Asp Ser Asp
   260 265 270
   Ser Trp Arg Ser Tyr Gln Asn Pro Leu Leu Phe Asp Asn Asn Gly Asn
   275 280 285
   Lys Lys Gln Ala Tyr Tyr Ala Val Leu Asp Ala Leu Asn Glu Gly Ser
   290 295 300
   Asp Asp Gly Gly Gly Pro Ser Asn Pro Pro Val Ser Pro Pro Pro Gly
   305 310 315 320
   Gly Gly Ser Gly Gln Ile Arg Gly Val Ala Ser Asn Arg Cys Ile Asp
   325 330 335
   Val Pro Asn Gly Asn Thr Ala Asp Gly Thr Gln Val Gln Leu Tyr Asp
   340 345 350
   Cys His Ser Gly Ser Asn Gln Gln Trp Thr Tyr Thr Ser Ser
   355 360 365
<210> 21
   <211> 4
   <212> PRT
   <213> Nonomuraea flexuosa
<220>
   <221> A synthetic linker sequence coding f or a Kex2- like pr ot ease cleavage signal Lys-Arg included in all the constructs to ensure cleavage of the fusion protein.
   <222> (1)..(4)
<400> 21
   Arg Asp Lys Arg
   1
<210> 22
   <211> 5
   <212> PRT
   <213> Nonomuraea flexuosa
<220>
   <221> An additional sequence preceding the Kex2 site in the expression cassette pALK1264, pALK1131 and pALK1134
   <222> (1)..(5)
<400> 22
   Gly Gln Cys Gly Gly
   1 5
<210> 23
   <211> 6
   <212> PRT
   <213> Nonomuraea flexuosa
<220>
   <221> The N-terminal sequence of mature Nf Xyn11A and recombinant Xyn11 A polypeptides, named as r 33. 4 kDa, r 23. 8 kDa and r 22. 0 kDa
   <222> (1)..(6)
<400> 23
   Asp Thr Thr Ile Thr Gln
   1 5
<210> 24
   <211> 6
   <212> DNA
   <213> Nonomuraea flexuosa
<220>
   <221> An Nr ul recognition site introduced into a Kex2 linker
   <222> (1)..(6)
<400> 24
   tcgcga 6
<210> 25
   <211> 12
   <212> DNA
   <213> Nonomuraea flexuosa
<220>
   <221> Kex2 linker which facilitates the construction of fusions
   <222> (1)..(12)
<400> 25 12
   cgcgacaagc gc 12

## Description

### Technical Field of the Invention

The present invention is related to molecular biology and particularly to methods and DNA constructs for increasing the production level in a filamentous fungal host when producing carbohydrate degrading (CD) enzymes, which in their native, unmodified state have a catalytic module (CAT) and a carbohydrate binding module (CBM) separated by a linker region. Carbohydrate degrading enzymes with the structure defined above are found among filamentous fungi and bacteria, such as strains of actinomycete, including *Nonomuraea flexuosa* Xyn11A or Xyn10A. For high yield production, a shortened DNA sequence, which encodes a truncated form of the desired carbohydrate degrading enzymes, is used.

### Background of the Invention

Plant cell walls consist mainly of a complex mixture of polysaccharides, primarily cellulose, lignin and hemicellulose. In most plant material, xylan is the major hemicellulose component, consisting of a main chain of 1,4-linked beta-D-xylopyranosyl residues that often carry acetyl, arabinosyl and glucuronosyl substituents. Carbohydrate degrading enzymes are useful as feed additives, because of their beneficial effects on the adsorption of feed components and in prebleaching of kraft pulp, wherein they are used as simple and cost-effective alternatives to toxic chlorine-containing chemicals. The main enzyme needed to enhance the delignification of kraft pulp is endo-β-1,4-xylanase (EC 3.2.1.8), but the presence of other enzymes such as mannanase, lipase, and α-galactosidase have been shown to improve the effect of enzymatic treatment. In enzyme-aided bleaching, pretreatment with xylanase removes xylan while preserving the cellulose content. Thereby, the need of bleaching chemicals is decreased and/or the brightness of the paper is increased. In feed applications, the beneficial effects obtained after enzyme addition, including increased growth rate and feed efficiency, result from the reduction of intestinal viscosity and release of nutrients from grain endosperm and aleurone layers.

The use of enzymatic treatments in both feed and pulp and paper industry has dramatically increased. As a corollary, the demand of carbohydrate degrading enzymes has also increased. This puts a pressure on the development of new efficient and cost-effective methods for the production of sufficient amounts of carbohydrate degrading enzymes having properties suitable for use in said industries. Xylanases that are active and stable at high temperature and alkaline pH are especially desirable in many industrial processes, due to the high temperatures and the alkaline conditions used in bleaching as well as the high temperatures used in downstream processing, e.g. pelleting.

Actinomycetes strains are known to produce thermostable enzymes with alkaline optima. Especially, thermophilic actinomycetes strains are a useful source of xylanases for industrial processes. Their activities and stability at high temperature are adequate for bleaching processes and other applications in which it is beneficial to perform the enzyme treatment at high temperatures. Useful genes have been cloned from e.g. *Thermomonospora fusca, Nonomuraea flexuosa* DSM43186, previously named as *Actinomadura flexuosa* or *Microtetraspora flexuosa* as well as from some *Streptomyces* species. The cloning of two *Nonomuraea flexuosa* xylanases has been described in US 5,935,836, US 6,300,114, US 6,506,593 and US 6,667,170.

The desired high temperature resistant carbohydrate degrading enzymes with extreme pH optima originate from relatively unstudied bacteria. Typically, they have low production levels and are unsuitable for industrial production in large scale. Little or practically no experience exists about fermentation of said bacteria. Accordingly, transfer of a gene originating from said microbes, and encoding the desired enzyme, into a heterologous host organism is a feasible alternative for producing the desired enzyme.

Bacterial enzymes have been produced in bacterial hosts and yeasts, as disclosed, for example, in US 5,306,633 and US 5,712,142. US 5,712,142 describes a method for increasing the thermostability of a bacterial cellulase from *Acidothermus cellulyticus* either by proteolytic cleavage or by expressing a shortened or truncated form of the gene encoding the full size cellulase in the yeast host, *Pichia pastoris.* WO 0196382 A2 describes a method for increasing the thermostability of *Rhodothermus marinus* cellulase by expressing a truncated form of the gene in *Escherichia coli.* Accordingly, several bacterial carbohydrate degrading enzymes have been described and it is known how to improve their thermostability.

Truncation of a multidomain xylanase from the anaerobic bacterium *Neocallimastix patriciarum* was shown to improve the expression in E. *coli,* as disclosed in WO 9325693 A1.

Filamentous fungi, including *Aspergillus, Trichoderma,* and *Penicillium*, are known as effective producers of homologous and heterologous proteins. They are by far the most preferred host organisms for large scale production of industrial enzymes, including bulk production of amylases, glucoamylases, cellulases, xylanases, etc. The first attempts to produce bacterial enzymes in filamentous fungi were discouraging. The yields of the bacterial enzymes were low, not exceeding a few tens of milligrams per liter. In many of the reports, the enzymes were detected only intracellularly (Jeenes, et al., Biotechnol. Genet. Eng. Rev., 9, 327-367, 1991; van den Hondel, et al., In J.W. Bennet and L.L. Lasure (ed.) More genetic manipulations in fungi: Academic Press, San Diego, Calif.).

Genetic fusion strategies have been developed and used in order to improve yields of heterologous proteins in filamentous fungi as disclosed in US 5,364,770 and WO 94/21785 and reviewed by Gouka et al., Appl. Microbiol. Biotechnol., 47, 1-11, 1997. Production of bacterial carbohydrate degrading enzymes from filamentous fungi, using gene fusions comprising a DNA sequence encoding a complete or a partial filamentous fungus secretable protein (polypeptide) as a carrier protein has been described in WO 97/27306 and US 2003/0148453. Using expression cassettes disclosed in said patent applications and comprising DNA sequences encoding a bacterial carbohydrate degrading enzyme fused in frame with a complete or partial filamentous fungus secretable protein, Paloheimo, et al., (Appl. Environ. Microbiol., 69, 7073-7082, 2003) have demonstrated that the production levels are remarkably increased when the gene encoding the bacterial enzyme is fused in frame with a filamentous fungal secretable polypeptide having an intact domain structure.

The objective of the present invention is to further improve the production levels of carbohydrate degrading enzymes, particularly, bacterial enzymes produced by recombinant DNA techniques from filamentous fungal hosts.

### The Summary of the Invention

The invention is related to a method and DNA constructs for improving the production levels of a carbohydrate degrading enzyme NfXyn11A using recombinant DNA techniques and filamentous fungi as hosts.

The carbohydrate degrading enzymes (CD) of the present invention is active on carbohydrate substrates and is an enzyme, wherein the complete enzyme in its original (native or unmodified) state has three characteristic domains or regions, which are a catalytic module (CAT) containing the active site and a carbohydrate binding module (CBM), which modules are separated by a linker region. Some carbohydrate binding modules, such as the carbohydrate binding module of AM50 (Nf Xyn10A) may have more than one sub-domain.

The method for obtaining the increased production level comprises a first step, wherein a DNA construct is designed. The DNA construct, which is introduced into a filamentous fungus host comprises regulatory sequences and a shortened DNA sequence encoding a truncated form of the carbohydrate degrading enzyme NfXyn11A, which comprises the catalytically active region of CAT and lacks part of the CBM module. The regulatory sequences are preferably derived from filamentous fungi and comprise at least one signal sequence.

The DNA sequence encoding the truncated form of the carbohydrate degrading enzyme originates from a bacterial strain, which is an actinomycetes strain *Nonomuraea.* Xylanase from *Nonomuraea flexuosa,* particularly, the thermostable xylanase NfXyn11A is an example of carbohydrate degrading enzymes, the yields of which may be increased by applying recombinant DNA techniques and shortened DNA sequences encoding the truncated target carbohydrate degrading enzyme, which lacks part or all of the CBM, or all of the CBM and part or all of the linker region.

Such DNA sequences may be natural sequences isolated from bacterial genomes. They may be synthetic sequences prepared by known methods, such as PCR. Synthetic sequences include the codon optimized DNA sequences, in which the nucleotide codons of natural bacterial DNA sequences are modified to resemble the codon usage in the filamentous fungus host. The truncated forms of the carbohydrate degrading enzyme encoded by such codon optimized sequences have the catalytic activity corresponding to the enzyme encoded by a natural DNA sequence.

The truncated form of said carbohydrate degrading enzyme is expressed under the control of regulatory sequences originating from the same or different filamentous fungi. The regulatory sequences comprise at least a signal sequence derived from a filamentous fungus. The yield can be even more improved when the carbohydrate degrading enzyme described above is expressed under the control of regulatory sequences consisting of promoter sequences, terminator sequences, and particularly, DNA sequences encoding a carrier protein (polypeptide) including a signal sequence. The regulatory sequences may be obtained from the same or different filamentous fungi and combined in arbitrary ways. This means that the origin of the regulatory sequences and their order may vary.

The DNA sequences encoding the carrier protein may either include the whole coding region of the mature secretable carbohydrate degrading protein or a selected region or domain thereof which in its native secretable form consists of the N-terminal catalytic module (CAT) and a C-terminal carbohydrate binding domain (CBD) separated by a linker (hinge) region. The carrier protein may include the catalytic module (CAT), or the catalytic module (CAT) and part or all of the linker (hinge) region, or the catalytic module (CAT) and all of the linker (hinge) region and part or all of the carbohydrate binding domain (CBD). *T. reesei* Man5A core region or Man5A core/hinge region (Stålbrand et al., Appl. Environ. Microbiol., 61: 1090-1097, 1995) are examples of such carrier proteins.

The secretable carbohydrate degrading protein may include only the catalytic domain (CAT) which can be used as a carrier polypeptide. *T. reesei,* XYNI and XYNII are examples of such proteins (Törrönen et al., Biotechnol., 10: 1461-1465). Some secretable proteins have their CBD in the N-terminal end of the enzyme. Example of such an enzyme is T. reesei Cel6A (CBHII) (Teeri et al., Gene, 51, 43-52, 1987). A Cel6A CBD consists of an A, an A+B or an A+B+B' regions, wherein A is a carbohydrate binding domain, B is a hinge (linker) region, and B' is a duplicated hinge (linker) region. These regions can be used alone or in different combinations as possible carrier polypeptides.

Signal sequences may be selected from various secretable filamentous fungal polypeptides, especially *Trichoderma* and *Aspergillus* signal sequences, including the Man5A signal sequence (Stålbrand et al., Appl. Environ. Microbiol., 61, 1090-1097, 1995) and the Cel6A signal sequence (Teeri et al., Gene, 51, 43-52, 1987).

Promoter sequences may be selected from various filamentous fungal promoters, especially from *Trichoderma* and *Aspergillus* promoters, including a strong *Trichoderma cel7A (cbh1)* promoter. Terminator sequences are also obtainable from filamentous fungi, especially preferred is a *Trichoderma cel7A* terminator sequence.

When the shortened DNA sequence is a shortened *Nf xyn11A* (*am24* (SEQ ID NO: 3) or *am24** (SEQ ID NO: 5) expressed and secreted solely under the control of a promoter and signal sequence originating from a filamentous fungi without any carrier protein or domains thereof, the production level of the truncated Nf Xyn11A of SEQ ID NO:12 is at least 2 times higher in shake flasks and often more than 8-10 times higher in a fermentation cultivation than the production level obtained when the corresponding full length *Nf xyn11A* is expressed and secreted under the control of the same promoter and signal sequence without a carrier protein or parts thereof.

When the shortened DNA sequence is a shortened *Nf xyn11A* (*am24* (SEQ ID NO: 3) or *am24** (SEQ ID NO: 5) expressed and secreted under the control of a promoter, signal sequence and a DNA sequence encoding a carrier protein (polypeptide) or domains thereof, all regulatory sequences originating from filamentous fungi, the production level of the truncated Nf Xyn11A of SEQ ID NO:12 is at least two times higher (in shake flasks) than with a DNA sequence encoding the corresponding full length Nf Xyn11A expressed and secreted under the control of the same promoter, signal and carrier protein sequence or domains thereof. Even higher production levels are obtained in fermentation cultivations. It is to be noted that a further improvement was still achieved when a DNA sequence encoding a truncated bacterial enzyme was used, even if an improved production level was already known to be achieved, when DNA sequences encoding a full length bacterial enzyme, were expressed and secreted under the control of intact domains of filamentous fungal secretable carrier protein (Paloheimo, et al., Appl. Environ. Microbiol., 69, 7073-7082, 2003).

### Sequence listing

- SEQ ID NO:1: *Nf xyn11A* nucleotide sequence (AJ508952), the coding region is from nt 303 to nt 1337. The GenBank sequence AJ508952 will show any revisions made to the sequence.
- SEQ ID NO:2: *am35* nucleotide sequence, *Nf xyn11A* coding region for the mature Nf Xyn11A (AM35) protein
- SEQ ID NO:3: *am24* nucleotide sequence, shortened form of *am35,* includes a STOP codon
- SEQ ID NO:4: *am35** nucleotide sequence, like *am35* but 9 codons are changed in the sequence (Example 10) (the changes do not alter the encoded amino acid sequence)
- SEQ ID NO:5: *am24** nucleotide sequence, like *am24* but 9 codons are changed in the sequence like in *am35** (Example 10)
- SEQ ID NO:6: *Nf xyn10A* nucleotide sequence (AJ508953), coding region is from nt 194 to nt 1672. The GenBank sequence AJ508953 will show any revisions made to the sequence.
- SEQ ID NO:7: *am50* nucleotide sequence, *Nf xyn10A* coding region for the mature Nf Xyn10A (AM50) protein
- SEQ ID NO:8: The nucleotide sequence encoding the AM50 core and linker regions, includes a STOP codon
- SEQ ID NO:9: The nucleotide sequence encoding the AM50 core region, includes a STOP codon
- SEQ ID NO:10: Nf Xyn11A amino acid sequence (AJ508952) encoded by the *Nf xyn11A* gene. The GenBank sequence AJ508952 will show any revisions made to the sequence.
- SEQ ID NO:11: r33.4 kDa = AM35 = amino acid sequence for the full length mature Nf Xyn11A protein encoded by *am35* and *am35** genes
- SEQ ID NO:12: AM24, amino acid sequence for the truncated form from AM35 encoded by *am24* and *am24** genes
- SEQ ID NO:13: r23.8 kDa, amino acid sequence for the truncated form from AM35
- SEQ ID NO:14: r22.0 kDa, amino acid sequence for the truncated form from AM35
- SEQ ID NO:15: Nf Xyn10A, the amino acid sequence (AJ508953) encoded by the *Nf xyn10A* gene. The GenBank sequence AJ508953 will show any revisions made to the sequence.
- SEQ ID NO:16: AM50, amino acid sequence for the full length mature Nf Xyn10A
- SEQ ID NO:17: AM50 core + linker, amino acid sequence for the truncated form from AM50
- SEQ ID NO:18: AM50 core, amino acid sequence for the truncated form from AM50
- SEQ ID NO:19: AM50 core + linker + α/β domains of the tail, amino acid sequence for the truncated form from AM50
- SEQ ID NO:20: AM50 core + linker + α domain of the tail, amino acid sequence for the truncated form from AM50
- SEQ ID NO:21: Amino acid sequence for the synthetic linker sequence coding for a Kex2-like protease cleavage signal Lys-Arg included in all the constructs to ensure cleavage of the fusion protein
- SEQ ID NO:22: An additional amino acid sequence preceding the Kex2 site in the expression cassette pALK1264, pALK1 131 and pALK1 134
- SEQ ID NO:23: The N-terminal amino acid sequence of mature Nf Xyn11A and recombinant Xyn11A polypeptides, named as r33.4 kDa, r23.8 kDa and r22.0 kDa
- SEQ ID NO:24: A nucleotide sequence for the *Nru*I recognition site introduced into a Kex2 linker
- SEQ ID NO:25: A nucleotide sequence for the Kex2 linker which facilitates the construction of fusions

### Short Description of the Drawings

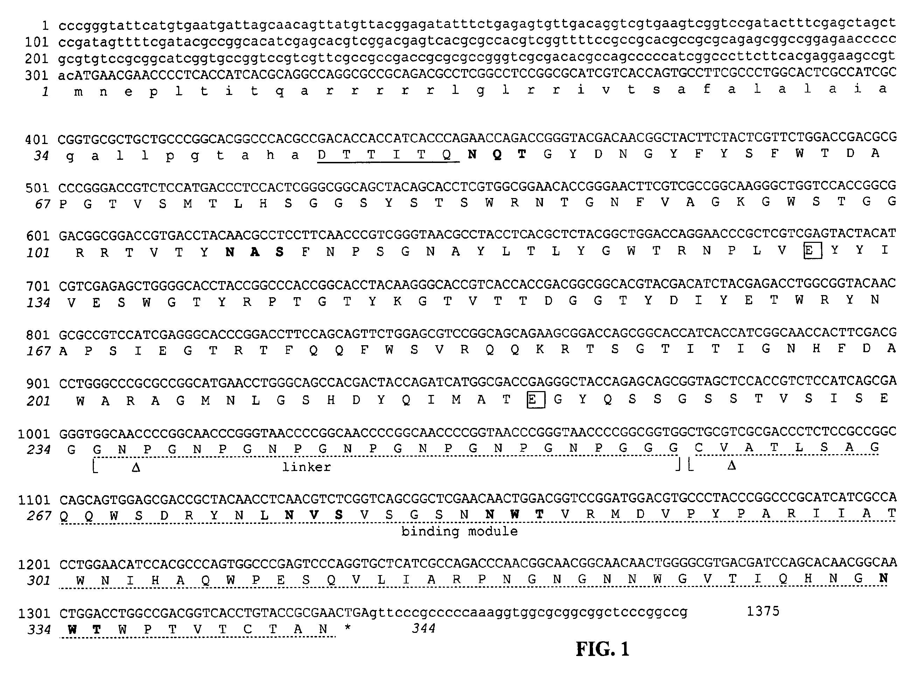

**Figure 1** shows the nucleotide sequence of the *Nonomuraea flexuosa xyn11A* gene and the deduced amino acid sequence. The stop codon is shown by an asterisk below the sequence. The mature N-terminal amino acid sequence, determined from the purified Xyn11A protein, is underlined. The active site glutamic acids are marked with an open box. The location of the linker and carbohydrate-binding module is indicated by a dotted line below the amino acid sequence. The cleavage sites of the r23.8 kDa and r22.0 kDa polypeptides are marked by a triangle. The sites for putative N-glycosylation in eukaryotic hosts are bolded.

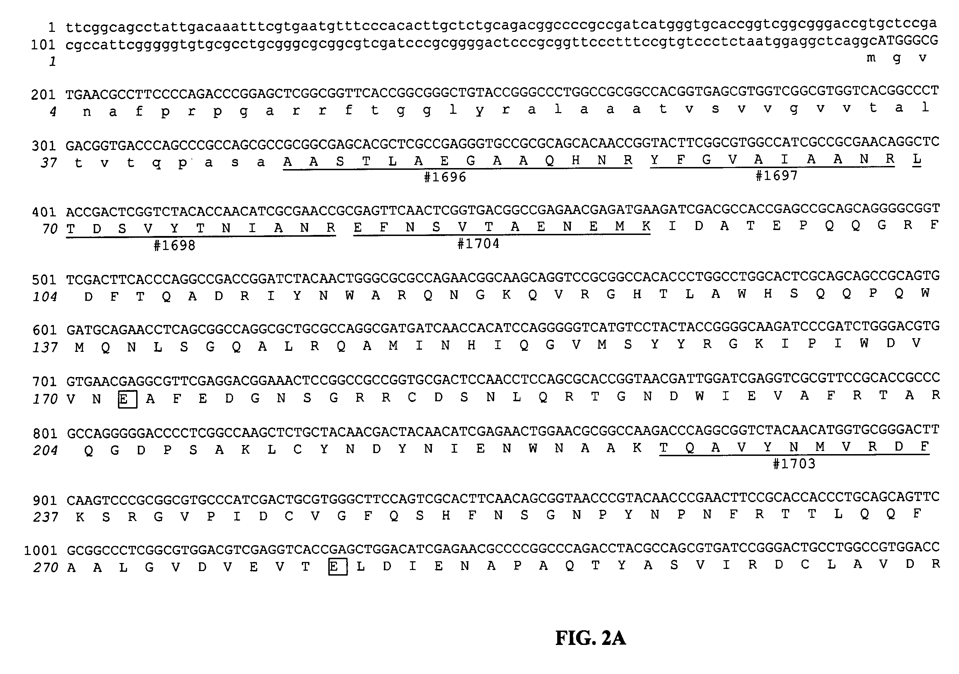

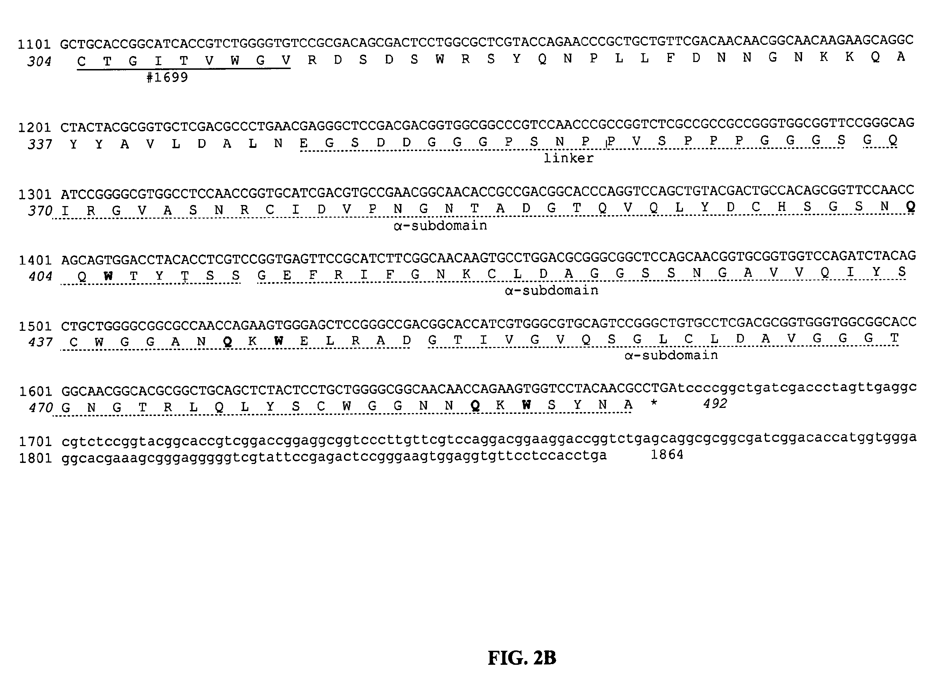

**Figure 2A** shows the nucleotide sequence of the *N. flexuosa xyn10A* gene and the deduced amino acid sequence. The stop codon is shown by an asterisk below the sequence. The tryptic peptide sequences, obtained from the purified Xyn10A protein, are shown by underlining below the amino acid sequence. The active site glutamic acids are marked with an open box. The putative location of the linker and carbohydrate-binding module, consisting of the α, β and γ subdomains (Fujimoto, et al., J. Mol. Biol., 300, 575-585, 2000) is indicated by a dotted line below the amino acid sequence. The QxW repeats present in the "ricin superfamily" CBMs (Fujimoto, et al., J. Mol. Biol., 300, 575-585, 2000; Hirabayashi, et al., J. Biol. Chem., 273, 14450-14460, 1998) are bolded. The nucleotide sequence continues in **Figure 2B****.**
**Figure 2B** shows the continuation of the nucleotide sequence depicted in **Figure 2A****.**

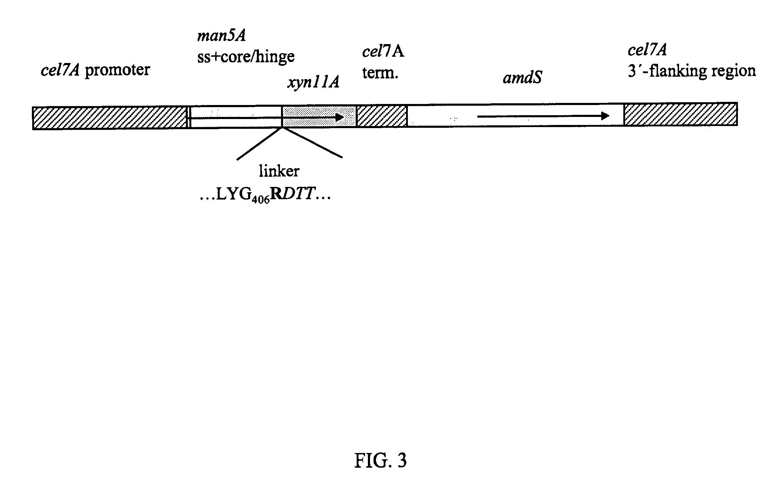

**Figure 3** shows the structure of the expression cassette pALK945, constructed to produce the recombinant mature *N. flexuosa* Xyn11A protein using *T*. *reesei* Man5A core/hinge as a carrier polypeptide. The gene fusion was expressed from *cel7A* promoter and termination of transcription was ensured by using *cel7A* terminator sequence. The *man5A* sequence included encoded the amino acids M₁ - G₄₀₆ (Stålbrand, et al., J. Biotechnol., 29, 229-242, 1993). The *amdS* gene (Kelly and Hynes, EMBO J., 4: 475-479) was included as a transformation marker and the *cel7A* 3'-flanking region was included, together with the *cel7A* promoter, to target the expression cassette into the *cel7A* locus by homologous recombination. Synthetic linker sequence coding for an additional Arg was included to ensure cleavage of the fusion protein. The amino acids coded by the *man5A* sequence are in regular font, those of *xyn11A* in italics and the synthetic amino acid for proteolytic cleavage is in bold.

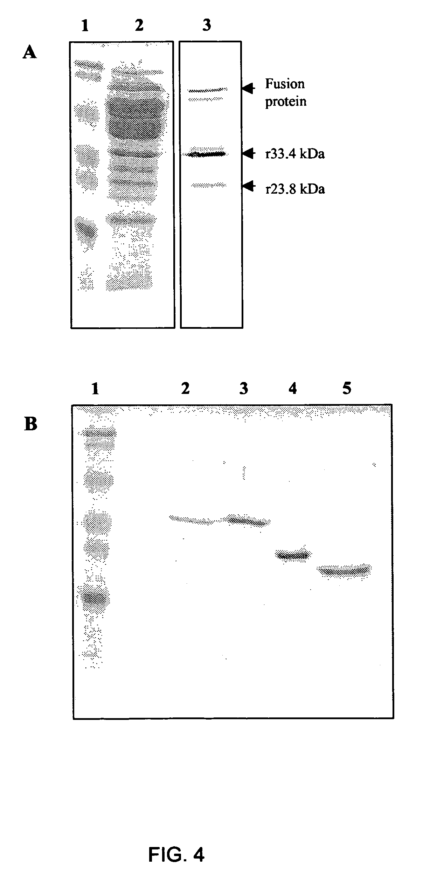

**Figure 4A** shows a sample of *T*. *reesei* cultivation medium from which the recombinant xylanase polypeptides (Example 7) were purified run on SDS-polyacrylamide gel after Coomassie Blue staining (lane 2) and after Western blot analysis (lane 3). The location of the r33.4 kDa and r23.8 kDa polypeptides and the fusion protein are shown by arrows. The r22.0 kDa form was not visible in the gel/Western blot shown, due to its low amount in the culture supernatant. Sizes of the molecular weight markers (on lane 1) were, from the top to the bottom: 104, 80, 46.9, 33.5, 28.3, 19.8 kDa. A polyclonal antibody made against the purified Nf Xyn11A was used for detection of the heterologous xylanase forms from the Western blot filter.
**Figure 4B** shows the samples of purified native Nf Xyn11A (lane 2), and the recombinant xylanase forms r33.4 kDa polypeptide (lane 3), r23.8 kDa polypeptide (lane 4) and r22.0 kDa polypeptide (lane 5) run on SDS-polyacrylamide gel and stained with Coomasie Blue. The molecular weight markers (lane 1) are the same as in **Figure 4A****.**

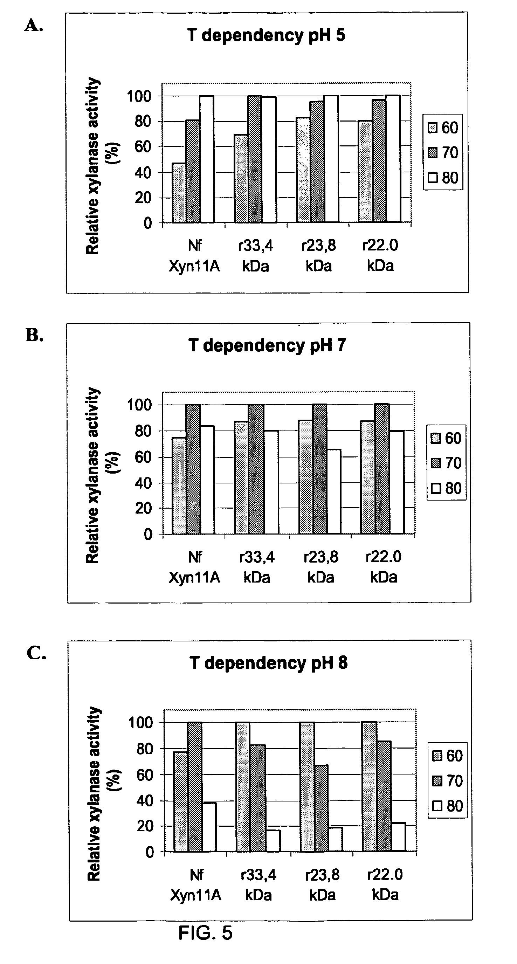

**Figure 5A** shows temperature profiles of the purified Xyn11A xylanases at pH 5. Effect of temperature was determined by incubation of the reaction mixture at pH 5 and different temperatures for 60 min. The relative (%) activity is expressed as percentage of maximum activity at the optimum temperature. The relative activities at pH 6 (not shown) were similar to those obtained at pH 5.
**Figure 5B** shows temperature profiles of the purified Xyn11A xylanases at pH 7. Effect of temperature was determined by incubation of the reaction mixture at pH 7 and different temperatures for 60 min. The relative (%) activity is expressed as percentage of maximum activity at the optimum temperature.
**Figure 5C** shows temperature profiles of the purified Xyn11A xylanases at pH 8. Effect of temperature was determined by incubation of the reaction mixture at pH 8 and different temperatures for 60 min. The relative (%) activity is expressed as percentage of maximum activity at the optimum temperature.

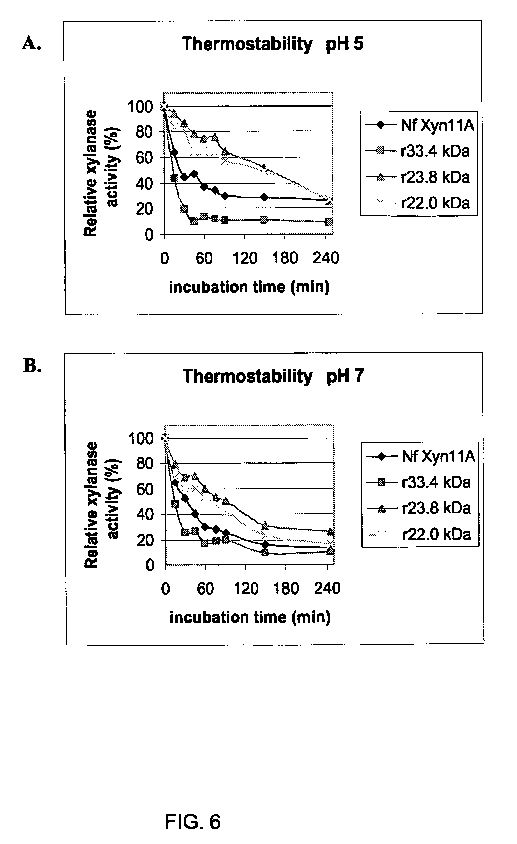

**Figure 6A** shows residual activity of the purified enzymes after incubation at 80°C at pH 5. Thermostability was determined by incubating the Xyn11A polypeptides at pH 5 for a period of 0, 15, 30, 45, 60, 75, 90, 150 and 245 min in the absence of substrate, after which the residual activities were measured at pH 7 and 70°C for 5 min.
**Figure 6B** shows residual activity of the purified enzymes after incubation at 80°C at pH 7. Thermostability was determined by incubating the Xyn11A polypeptides at pH 7 for a period of 0, 15, 30, 45, 60, 75, 90, 150 and 245 min in the absence of substrate, after which the residual activities were measured at pH 7 and 70°C for 5 min.

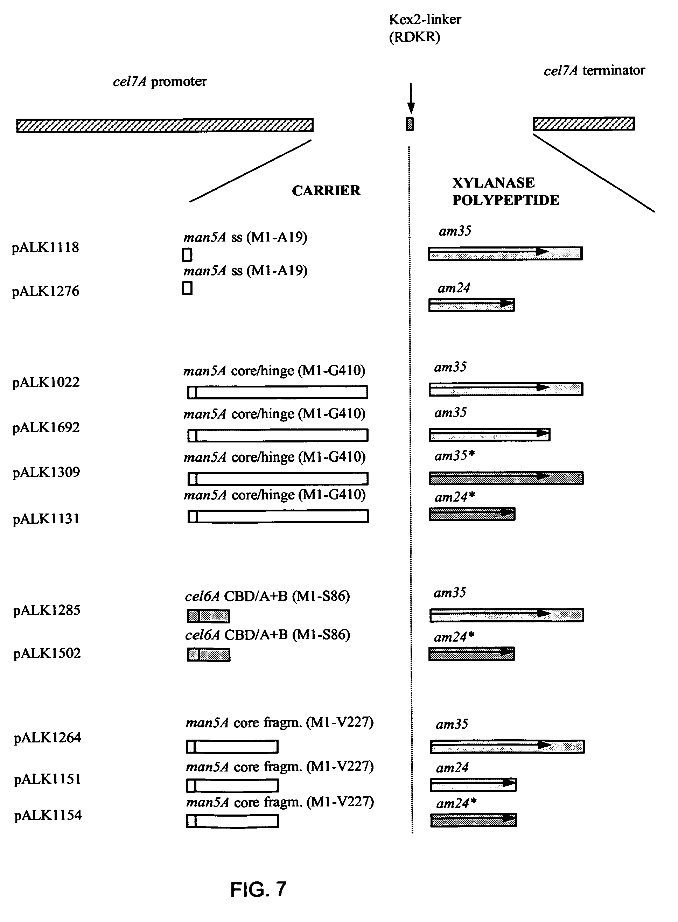

**Figure 7** shows the overall structure of the expression cassettes constructed to study the effect of the truncation of the *am35* gene on xylanase production levels in *Trichoderma reesei.* The gene fusions were expressed from *cel7A* promoter and termination of transcription was ensured by using *cel7A* terminator sequence. The carrier polypeptides used were encoded either by the *man5A* core/hinge sequence (M₁-G₄₁₀ in pALK1022, pALK1692, pALK1309 and pALK1131), the *cel6A* CBD sequence (M₁-S₈₆) in pALK1285 and pALK1502 or the *man5A* sequence encoding for a fragment of the Man5A core (M₁-V₂₂₇ in pALK1264, pALK1151 and pALK114). The Cel6A CBD block A codes for the tail of the protein and B for the hinge region. In the plasmids pALK1118 and pALK1276 the xylanase gene was fused to the *man5A* signal sequence. A synthetic linker sequence coding for a Kex2-like protease cleavage signal Lys-Arg (included as RDKR (SEQ ID NO:18)) was included in all the constructs to ensure cleavage of the fusion protein. The expression cassette pALK1264, pALK1131 and pALK1134 also included an additional sequence coding for GQCGG (SEQ ID NO:19) preceding the Kex2 site. The amino acids coded by *am35* (SEQ ID NO: 2) and *am35** (SEQ ID NO: 4) are D₄₄-N₃₄₄ (the full length mature protein, Fig. 1; SEQ ID NO: 11) and by the truncated *am35*/*am35** sequences (*am24* (SEQ ID NO: 3) and *am24**; SEQ ID NO: 5) are D₄₄-L₂₆₃ (Fig. 1; SEQ ID NO: 12). In *am35** (SEQ ID NO: 4) and *am24** (SEQ ID NO: 5) nine codons have been changed as described in Example 10. The changes do not alter the encoded amino acid sequences. The *amdS* marker gene and the *cel7A* 3'-flanking region (to target the expression cassette into the *cel7A* locus by homologous recombination) were included in all the constructs after the *cel7A* terminator sequence (identically to the pALK945 expression cassette shown in Fig. 3).

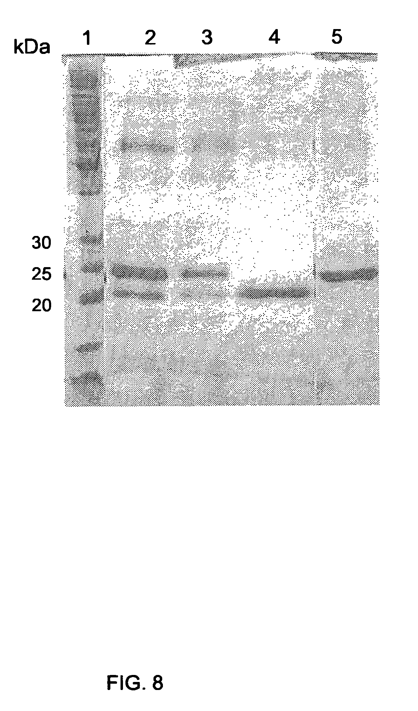

**Figure 8** shows a Coomassie Blue stained SDS-polyacrylamide gel, in which a sample of the culture supernatant of a *T*. *reesei* transformant producing the truncated form of Nf Xyn11A xylanase (AM24) was run. The Ce16A CBD was used as a carrier polypeptide. The purified r23.8 and r22.0 kDa forms were run in parallel on the gel. Lanes: 1. A molecular weight marker (the molecular mass of the relevant marker proteins is shown), 2. and 3. Culture supernatant from a *T*. *reesei* transformants producing the truncated Xyn11A, 3. Purified r22.0 kDa xylanase, 5. Purified r23.8 kDa xylanase.

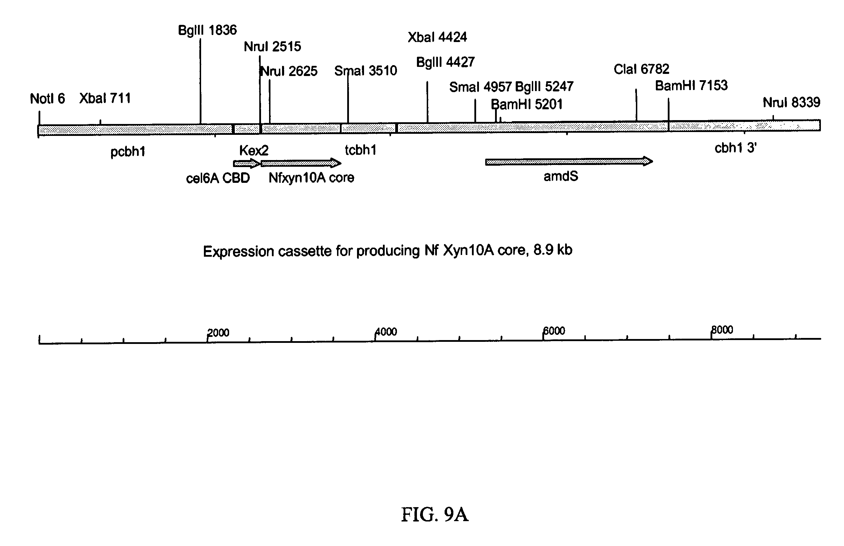

**Figure 9A** shows the expression cassette for producing a truncated form of Nf Xyn10A, the Nf Xyn10A core, in *T*. *reesei.* The core includes the amino acids A₄₅₋N₃₄₅ (Fig. 2; SEQ ID NO: 18). The *cel7A* promoter, *cel6A* signal peptide and *cel7A* terminator sequences are used (as in the construct pALK1285 and pALK1502, Fig. 7) for Nf Xyn10A production. The Cel6 CBD (A+B) is used as a carrier polypeptide (as in the constructs pALK1285 and pALK1502, Fig. 7). A sequence encoding a Kex2 site (RDKR (SEQ ID NO:18)) is included between the carrier and the xylanase sequences. The *amds* marker and the *cbh1* 3'-fragment are included as in the constructs for producing Nf Xyn11A.

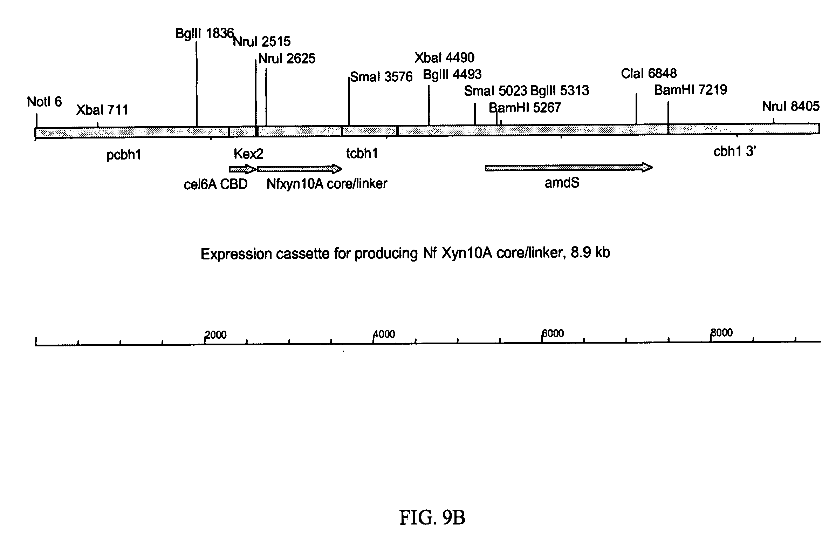

**Figure 9B** shows the expression cassette for producing a truncated form of Nf Xyn10A, the Nf Xyn10A core/linker in *T*. *reesei.* The core/linker includes the amino acids A₄₅-S₃₆₇ (Fig. 2; SEQ ID NO: 17). The other sequences (*cel7A* promoter, *cel6A* signal peptide, *cel6A* CBD encoding sequence, *cel7A* terminator, the sequence encoding the Kex2 site, the *amdS* marker and the *cbh1* 3'-fragment) are as in the constructs in Fig. 9A.

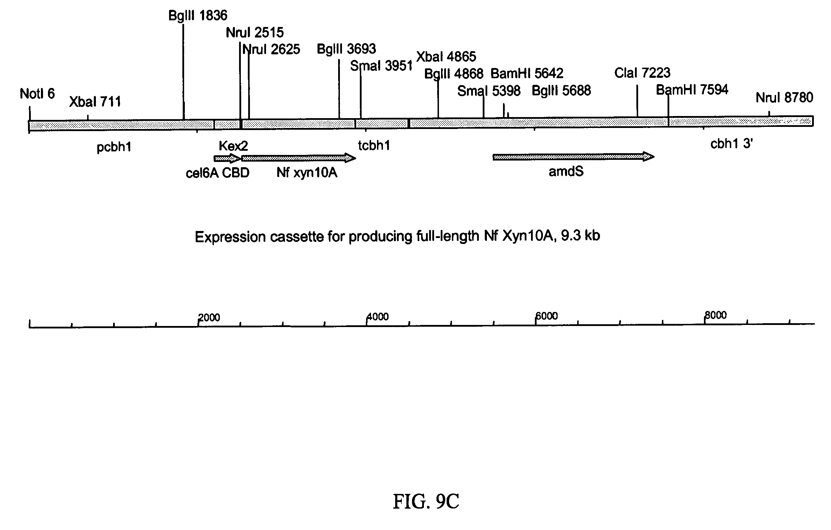

**Figure 9C** shows the expression cassette for producing the full-length Nf Xyn10A in *T*. *reesei.* The full-length xylanase includes the Nf Xyn10A amino acids A₄₅-A₄₉₂ (the full-length protein from the N-terminal end, Fig. 2; SEQ ID NO: 16). The other sequences (*cel7A* promoter, *cel6A* signal peptide, *cel6A* CBD encoding sequence, *cel7A* terminator, the sequence encoding the Kex2 site, the *amdS* marker and the *cbh1* 3'-fragment) are as in the constructs in Fig. 9A.

### List of nucleotide sequence accession numbers

- AJ508952: *xyn11A* nucleotide sequence of *N.flexuosa* DSM43186
- AJ508953: *xyn10A* nucleotide sequence of *N.flexuosa* DSM43186

### Donor organisms

*Nonomuraea flexuosa* DSM43186 (ATCC35864)

### General Description of the Invention

### Abbreviations and Nomenclature

- Nf: *Nonomuraea flexuosa*
- *xyn10A*: gene for family 10 xylanase
- Xyn10A: protein for family 10 xylanase
- *xyn11A*: gene for family 11 xylanase
- Xyn11A: protein for family 11 xylanase
- CBM, CBD: carbohydrate binding module/domain, Partial structure of carbohydrate degrading enzyme
- CBM: carbohydrate binding module herein the term CBM is used for truncated carbohydrate degrading enzymes
- CBD: carbohydrate binding domain herein the term CBD is used for domains of carrier protein
- *cel7A* (*cbh1*): cellobiohydrolase 1 gene in *Trichoderma reesei*
- *cel7B* (*egl1*): endoglucanase 1 gene of *Trichoderma reesei*
- *cel6A* (*cbh2*): cellobiohydrolase 2 gene of *Trichoderma reesei*
- *cel5A* (*egl*2): endoglucanase 2 gene of *Trichoderma reesei*
- *man5A*: mannanase gene of *Trichoderma reesei*
- *Nf xyn11A*: *N. flexuosa* xylanase gene from the family 11
- *Nf xyn10A*: *N. flexuosa* xylanase gene from the family 10
- *am35*: *Nf xyn11A* gene encoding the full-length form of the Nf Xyn11A mature protein (amino acids D₄₄ - N₃₄₄)
- *am24*: shortened form of *Nf xyn11A* encoding the truncated form of the Nf Xyn11A mature protein (amino acids D₄₄ - L₂₆₃)
- *am35**: *am35* but including the following changes in codons: Gly₅₃ GGG to GGC, Ala₆₆ GCG to GCC, Gly₆₈ GGG to GGC, Arg₈₅ CGG to CGC, Gly₈₈ GGG to GGC, Gly₁₀₀ GGA to GGC, Arg₁₀₁ CGG to CGC, Arg₁₀₂ CGG to CGC and Val₁₀₄ GTG to GTC
- *am24**: *am24* but including the same changes in codons as *am35**
- *am50*: *Nf xyn10A* gene encoding the full-length form of the Nf Xyn10A mature protein (amino acids A₄₅ - A4₄₉₂)
- AM35: same as r33.4 kDa, see below, encoded by *am35* and *am35**
- AM24: truncated form from AM35, encoded by *am24* and *am24** (amino acids D₄₄ - L₂₆₃)
- r33.4 kDa: full-length recombinant Nf Xyn11A mature protein (amino acids D₄₄ - N₃₄₄)
- r23.8 kDa: cleavage form of the full-length recombinant Nf Xyn11A mature protein (amino acids D₄₄ - V₂₆₀)
- r22.0 kDa: cleavage form of the full-length recombinant Nf Xyn11A mature protein (amino acids D₄₄ - N₂₃₅)
- AM50: full-length recombinant Nf Xyn10A mature protein (amino acids A₄₅ - A₄₉₂), encoded by *am50*.

### Classification of Carbohydrate-Active Enzymes

Information about the nomenclature of carbohydrated degrading enzymes including cellulases, xylanases, mannanases, and others can be found at Coutinho, P.M.& Henrissat, B. (1999) Carbohydrate-Active Enzymes server at URL:
http://afmb.cnrs-mrs.fr/CAZY/.

### General Description of the Invention

### Terminology

In the present invention most definitions have the same meaning they generally have in the corresponding scientific fields. Some terms are used in somewhat different way. Therefore, they are explained in more detail below.

The term **"carbohydrate degrading (CD) enzyme"** means an enzyme active on carbohydrate substrate and having a catalytic module (CAT) and a carbohydrate binding module (CBM) separated by a linker region. The terms "domain" or "region" are also used for the term "module". Bacterial carbohydrate degrading enzymes are obtainable from several different bacterial strains, including actinomycete strains of *Nonomuraea, Thermomonospora,* particularly *Nonomuraea flexuosa* or *Thermomonospora fusca.* The method seems to be applicable to enzymes obtainable from some other microorganisms, for example, xylanases obtainable from *Chaetomium,* particularly *C*. *thermophilum,* which is a filamentous fungus. Carbohydrate degrading enzymes include cellulose and hemicellulose degrading enzymes, particularly some specific mannan degrading enzymes. Particularly useful are thermostable xylanases including Nf Xyn10A or Nf Xyn11A. Information about carbohydrate degrading enzymes and their structures are found for example in Gilkes, et al., Eur. J. Biochem., 202, 367-377. 1991, Teeri, et al., J. Biotechnol., 24, 169-176, 1992, Stålbrand, et al., Appl. Environ. Microbiol., 61, 1090-1097, 1995, Tomme, et al., (1995) Enzymatic Degradation of Insoluble Polysacharides (Saddler & Penner, Eds.) Cellulose-binding domains: classification and properties. pp 142-163, American Chemical Society, Washington). In most carbohydrate degrading enzymes the CBM is situated in the C-terminal end of the molecule, but some enzymes, for example *T*. *reesei* Ce16 (CBHII) and Ce15A (EGII) have their CBM in the N-terminal end of the enzyme. The provision of higher production levels by removal of the CBM or part of it or the CBM and the linker or part of it of the bacterial carbohydrate degrading enzyme is believed to work even if the CBM is not situated in the C-terminal end of carbohydrate degrading enzyme used to exemplify the invention.

The term **"truncated form of the carbohydrate degrading enzyme"** means an enzyme, particularly a bacterial enzyme, for example, from an actinomycetes strain.which lacks part of the amino acid sequence. Typically, this means that a part or all of the CBM, or alternatively all of the CBM and a part or all linker region is missing from the truncated form. Truncated form of the enzyme contains an intact catalytic domain or at least a region of the catalytic domain which is essential in catalytic activity of the carbohydrate degrading enzyme (CD). The truncated enzyme is expressed and secreted under the control of suitable regulatory sequences, including promoters and signal sequences.

The term "catalytically active region of CAT" means that the region carries at least the enzyme active site, which provides the specificity for its particular substrate and contributes the amino acid residues that directly participate in the making and breaking of chemical bonds. The active site amino acid residues are conserved within different CD families. The active sites of family 10 and family 11 endo -1,4-xylanases, for example, contain two conserved glutamic acids, which are essential for catalytic activity. The catalytically active region of CAT has similar catalytic activity than the corresponding intact CAT or the intact carbohydrate degrading enzyme and can be measured, for example, with enzyme activity assays.

The term **"DNA construct"** means an expression or transformation construct. The DNA construct comprises at least a shortened DNA sequence, which originates from a bacterium and encodes a shortened form of said bacterial carbohydrate degrading enzyme preferably in combination with appropriate regulatory sequences, which include a promoter, a signal sequence with or without a carrier sequence, and a terminator sequence originating from a filamentous fungus. The DNA construct includes at least the DNA sequences, which are essential for competent expression and secretion of the shortened form of the desired carbohydrate degrading enzyme. The DNA constructs can be provided in two different forms, as an expression cassette or an expression plasmid.

In an expression plasmid the DNA construct may further contain plasmid elements and reporter gene sequences for replication and selection in *E. coli*. An expression cassette favourably consists of the DNA sequences, which are essential for the expression and secretion of the bacterial enzymes in filamentous fungi. The expression cassette does not include plasmid elements and reporter sequences. The selection marker can be included in either the expression plasmid/cassette or it can be separately transformed to the filamentous fungal host by using co-transformation method. In other words, plasmid elements and reporter sequences are removed from the expression plasmids in order to obtain the expression cassettes for transformation. However, both forms may include and preferably include sequences, which enable locus targeted transformation in the filamentous fungal host. The DNA construct may thereby be targeted to a selected locus in the genome of the host.

The term **"regulatory sequences"** means DNA sequences controlling the expression and secretion of the carbohydrate degrading enzyme having the structure defined above. The regulatory sequences used in the present invention preferably originate from filamentous fungi, such as *Aspergillus* and *Trichoderma,* particularly *Trichoderma reesei.*

The regulatory sequences comprise at least one signal sequence derived from a filamentous fungus, and also at least one promoter from a filamentous fungus, including the strong *Trichoderma cel7A (cbh1)* promoter. In a preferred embodiment of the invention the shortened DNA sequence encoding the truncated form of the carbohydrate degrading enzyme, is fused in frame with a DNA sequence encoding a carrier protein (polypeptide). The carrier protein is preferably encoded by DNA sequences, which may be obtained from and put together of domains derived from the same or different filamentous fungi and encode one or more, preferably intact domains of a filamentous fungal secretable enzyme. The structure of an intact domain resembles the corresponding region in the full length enzyme due to correct folding of the primary amino acid sequence. Therefore, secretion pathways of carrier polypeptides containing one or more intact domains of the filamentous fungal secretable enzyme are expected to be similar to the native full length enzyme. Applicable, secretable enzymes may comprise a core domain containing the active or catalytic site of the enzyme and a substrate binding domain or carbohydrate binding domain (CBD) linked by a hinge region, but the carrier protein may also be a secretable enzyme naturally consisting solely of the core region, as exemplified by certain filamentous fungal xylanases, e.g. *T*. *reesei* XYNI and XYNII. An intact CBD or CBD and linker may also be used in order to achieve a high production level. Different domains or regions of filamentous secretable enzymes derived from different filamentous fungi may be put together in varying and arbitrary order. The DNA sequences encoding the regulatory sequences including the carrier protein may be constructed by combining DNA sequences derived from different filamentous fungal sources.

The most preferred combinations include constructs, wherein the carrier protein is a *T*. *reesei* Man5A core or Man5A core/hinge region, or a *T*. *reesei* Cel6A CBD (A, A+B or A+B+B'), wherein A means the substrate binding domain or tail; B means the linker (hinge) region; and B' means a duplicated linker(hinge) region. Said DNA sequences encoding the carrier polypeptide are preferably combined with a *cel7A (cbh1)* promoter. It is possible to construct a multitude of other combinations. The invention includes but is not limited to the following domains: *Trichoderma* CBDs from Cel6A (CBHII), and Ce15A (EGII) carrying a CBD domain in their N-terminal end, xylanase *Trichoderma* (XYNI, XYNII) and *Trichoderma* CBDs from Ce17A (CBHI), Ce17B (EGI) Ce161A (EGIV), Ce145 (EGV) and Ce174 (EGVI) carrying a CBD in their C-terminal end. *Aspergillus* proteins/polypeptides encoded by *glaA* (glucoamylase) and *Penicillium* proteins/polypeptides encoded by *xynA* (Alcocer, et al., Appl. Microbiol. Biotechnol., 60, 726-732, 2003) and the corresponding structures from other organisms.

Some other filamentous fungus secretable proteins having applicable domains are found among the carrier polypeptides described in Gouka, et al., Appl. Microbiol. Biotechnol., 47, 1-11, 1997.

The most preferred combinations include the Man5A core or core/hinge region as a carrier polypeptide, the Kex-2 linker (SEQ ID NO: 21) with or without the additional sequence (SEQ ID NO: 22) preceeding the Kex-2 linker and the truncated form of AM35 polypeptide, i.e. AM24 (SEQ ID NO: 12).

The most preferred combinations further include the Ce16A CBD (A, A+B or A+B+B') region as a carrier polypeptide, the Kex-2 linker (SEQ ID NO: 21) with or without the additional sequence (SEQ ID NO:22) preceeding the Kex-2 linker and the truncated form of AM35 polypeptide, i.e. AM24 (SEQ ID NO: 12).

In the present invention the term **"filamentous fungus"** is used in several connections including host organism and donor organism for regulatory sequences. The preferred filamentous fungi, include any transformable filamentous fungi in which expression can be achieved, most preferably they include but are not limited to *Trichoderma, Aspergillus,* and *Penicillium* strains. Particularly preferred hosts and donor organisms are *Trichoderma reesei, Trichoderma longibrachiatum, Trichoderma viridae, Trichoderma koningii, Aspergillus niger, Aspergillus sp., Penicillium sp., Humicola sp.,* including *Humicola insolens, Chrysosporium sp*., *Fusarium sp*., *Hypocrea sp.* and *Emericella sp*., etc.

The term **"an increased production level"** means that the production level of the truncated form of the bacterial carbohydrate degrading enzyme is higher than the production level of the full length enzyme in a similar construct which is otherwise identical but differs in the length of the DNA sequence encoding the bacterial enzyme. The increase in production level is in this case measured as an increase of activity (Table 4), because the increase in activity in this case is independent of the specific activity, as demonstrated by the results shown in Table 1. It is to be noted that the increase in efficacy or in the production level is measured as xylanase activity from the culture media of isogenic single-copy transformants or host cells containing only one copy of the DNA construct in question. Furthermore, the isogenic single copy is located in the same locus, for example in the *cbh1* locus. Therefore, it is unambiguously excluded that the increase in production level would be caused by introduction of multiple copies of the DNA construct or of differences in the site of integration. The transformants analysed differ only in that the DNA constructs of the present invention comprise a shortened DNA sequence encoding a truncated form of the full length carbohydrate degrading enzyme NfXyn11A, whereas the prior art DNA construct comprises a DNA sequence encoding the corresponding full length enzyme. The production levels are accordingly based upon unambigous comparisons.

### The Detailed Description of the Invention

In the present invention it is demonstrated that production level-of an carbohydrate degrading (CD) enzyme, particularly the bacterial enzyme NfXyn11A originating from *Nonomuraea flexuosa*, produced in a *Trichoderma reesei* host may be increased not only by fusion strategies as described by Paloheimo, et al., Appl. Environ. Microb., 69, 7073-7082, 2003, but also by using truncated DNA sequences encoding a truncated NfXyn11A of SEQ ID NO:12. By expressing said shortened DNA sequences yields of the truncated carbohydrate degrading enzyme NfXyn11A increase. The function, including specific activity of the truncated enzyme is the same or similar to that obtained with the corresponding full-length enzyme. The preparations obtained with the method and constructs of the present invention are useful in industrial processes requiring high temperatures and pHs.

In the following examples the invention is described in more detail.

### Example 1

### Methods used in the analysis and characterisation of proteins (polypeptides)

### a. Protein and enzyme assays

Protein concentrations in the culture media and purified enzyme samples were assayed after TCA precipitation by the method of Lowry, et al. (J. Biol. Chem., 193, 265-275, 1951) using bovine serum albumin as a standard protein. During enzyme purifications, proteins were monitored at 280 nm. Xylanase activity was assayed by using 1% (w/v) birch xylan (Roth no. 7500, Roth, Karlsruhe, Germany) as a substrate in 50 mM Mcllvaine citrate-phosphate buffer according to the method of Bailey, *et al*., (Enzyme Microb. Technol., **3**:, 53-157, 1992). During enzyme purification and determination of the specific activity of the pure proteins the assay was performed at pH 7, 60°C for 5 min, otherwise as stated in the figure legends. For characterization of the purified Xyn11A polypeptides the buffer was supplemented with 0.1 % BSA, except for the determination of thermostability which was performed without BSA.

### b. Protein electrophoresis

The samples were run on 12% polyacrylamide slab gels containing 0.1% SDS on a Mini Protean II electrophoresis system (Bio-Rad Laboratories, Inc., Hercules, Calif.) and stained with Coomassie Brilliant Blue R250. Detection of the xylanase and xylanase polypeptides on the Western blot filters was carried out with a polyclonal rabbit antibody raised against the purified Nf Xyn11A xylanase prepared at Diabor Ltd. (Oulu, Finland) and the Protoblot AP System (Promega Corp., Madison, Wisc.).

### c. Preparation and sequencing of peptides

Purified fractions containing xylanase activity were subjected to tryptic digestion as described in Fagerström and Kalkkinen (Biotechnol. Appl. Biochem., 21, 223-231, 1995). Sequencing was performed by Edman degradation in a gas-pulsed-liquid-phase sequencer (Kalkkinen and Tilgmann, J. Protein Chem., 7, 242-243, 1988) and the phenylthiohydantoin amino acids were analyzed on-line by using narrow bore reverse phase HPLC. For N-terminal sequencing, SDS-PAGE gels were blotted on a PVDF filter and the protein spots were directly subjected to Edman degradation as above. The sequencing was performed at the Institute of Biotechnology (Helsinki, Finland). The C-termini of the purified recombinant polypeptides were determined at the Protein Analysis Center at the Karolinska Institutet (Stockholm, Sweden).

### d. Estimation of pI and determination of the molecular mass

The pI of the purified xylanases was estimated by chromatofocusing on a 4 ml prepacked Mono P HR 5/20 column (Amersham Biosciences) equilibrated with 75 mM Tris- CH ₃COOH (pH 9.5) and eluted with 10% Polybuffer 96 (Amersham Biosciences) in 75 mM Tris-CH₃COOH (pH 6.3). The pH was measured from the fraction containing xylanase activity. Molecular masses of the purified xylanases were determined by Bruker Biflex Reflector MALDI-TOF mass spectrometer (Bruker Daltonik GmbH, Bremen, Germany).

### Example 2

### DNA techniques used in constructing plasmids and strains

Standard DNA methods (Sambrook, et al., Molecular cloning: a laboratory manual, 2nd ed. Cold Spring Harbor Laboratory, Cold Spring Harbor, 1989) were used in constructing plasmids, transforming E. *coli* and performing Southern blots. Each enzyme and kit was used according to the instructions from the supplier. The enzymes for DNA modifications were purchased from Roche Diagnostics GmbH (Mannheim, Germany), New England Biolabs (Beverly, Mass.) and Finnzymes (Espoo, Finland). Qiagen columns (Qiagen GmbH, Hilden, Germany) or Magic Miniprep kits (Promega, Madison, Wisc.) were used in the plasmid isolations. The oligonucleotides were either synthesized using ABI 381A DNA synthesizer or ordered from Sigma-Genosys. The sequencing reactions were analysed either by using ABI 373A or ABI Prism™ 310 Genetic Analyzer (Applied Biosystems, Foster City, Calif.). Polymerase chain reactions (PCR) were performed using PTC-100 Programmable Thermal Controller (MJ Research Inc, Watertown, Mass.). DNA fragments for subcloning and transformations were isolated from low melting point agarose gels (BioWhittaker Molecular Applications Inc., Rockland, Maine) by the freeze-thaw-phenol method (Benson, BioTechniques, 2, 66-68, 1984), by using β-agarase (New England Biolabs) or by using the Qiaex II Gel Extraction Kit (Qiagen GmbH).

The genomic DNAs were isolated as described in (Raeder and Broda, Lett. Appl. Microbiol., 1, 17-20, 1985). Digoxigenin labeled (Roche Diagnostics GmbH) expression cassettes were used as probes in the Southern blot hybridizations.

### Example 3

### Microbial strains used as hosts and in constructing plasmids, growth media and growth conditions

Plasmids were propagated in *Escherichia coli* XL1-Blue or XL10-Gold (Stratagene, La Jolla, Calif.). The vector backbones used in the plasmid constructions were pUC18 (EMBL Database Accession No L09136), pUC19 (L09137), pBluescript SK- and pBluescript II KS + or KS-(Stratagene). All E. *coli* cultivations were performed over night at 37 °C in Luria-Bertani medium into which ampicillin had been added (50 µg/ml).

The *Trichoderma reesei* strains ALKO3620 and ALKO4468 were used as parents for the transformations. *T. reesei* ALKO3620 is an endoglucanase II-negative strain. It was constructed from the low protease mutant strain ALKO2221, derived from the strain VTT-D-79125 (Bailey and Nevalainen, Enzyme Microb. Technol., 3, 153-157, 1981) by UV-mutagenesis (Mäntylä, et al., Abstr. 2nd Eur. Conf. Fungal Genetics, abstr. B52, 1994) as follows. The endoglucanase 2 gene *(cel5A* or *egl2*, originally named as *egl3*; (Saloheimo, et al., Gene 63, 11-21, 1988)) was replaced by the phleomycin resistance-encoding marker gene from *Streptoalloteichus hindustanus*, Sh *ble* (Drocourt, et al., Nucleic Acids Res., 18, 4009, 1990). The 3.3 kb *Bgl*II-*Xba*I fragment from the plasmid pAN8-1 (Matheucci, et al., Gene, 8, 103-106, 1995), containing the *ble* gene flanked by the *Aspergillus nidulans gpd* promoter and *trpC* terminator was used. The *cel5A* flanking sequences in the replacement cassette (the 5'-region being the 1.4 kb *Xho*I - *Sac*I fragment about 2.2 kb upstream from the *cel5A* gene and the 3'-region the 1.6 kb *Avr*II - *Sma*I fragment about 0.2 kb from the end of the *cel5A* gene) were isolated from the *egl3* λ clone (Saloheimo, et al., Gene, 63, 11-21, 1988). The strategy for the replacement was as described in (Suominen, et al., Mol. Gen. Genet., 241, 523-530, 1993). *T*. *reesei* ALKO4468 is an endoglucanase I and II-negative strain. It was constructed from the strain ALKO3620 by further replacing the endoglucanase 1 gene, *cel7B* (*egl1,* Penttilä, et al., Gene, 45, 253-263, 1986), by the E. *coli* hygromycin B phosphotransferase gene, *hph* (Gritz, et al., Gene, 45, 179-188, 1983), conferring resistance to hygromycin B. The 1.7 kb *Not*I*-Nsi*I fragment from the plasmid pRLM_{EX}30 (Mach, et al., Curr. Genet., 25, 567-570, 1994) was used in which of the *hph* gene is expressed from the *T. reesei* pyruvate kinase (pki) promoter and the transcription is terminated using the *cel6A* terminator sequences. The plasmid pRLM_{EX}30 was kindly provided by Prof. Christian P. Kubicek (Institut für Biochemische Technologie, TU Wien, Austria). The *cel7B* flanking regions were as described in (Suominen, et al., Mol. Gen. Genet., 241, 523-530, 1993). The single-copy replacements of the *cel5A* and *cel7B* genes by the marker genes in *T. reesei* ALKO3620 and ALKO4468 were verified by Southern blot analysis, as described in (Suominen, et al., Mol. Gen. Genet., 241, 523-530, 1993).

*T*. *reesei* strains were sporulated on PD agar slants (Potato Dextrose Broth, Difco, Detroit, Mis.). The transformants were selected on *Trichoderma* minimal medium containing acetamide as a nitrogen source (Penttilä, et al., Gene, 61, 155-164, 1987). The fungal mycelia for DNA isolations were obtained after growing the strains for two days on *Trichoderma* minimal medium containing 2% proteose peptone (Difco). Complex lactose-based cellulase-inducing media (Joutsjoki, et al., Curr. Genet., 24, 223-228, 1993) were used for enzyme production in shake flasks and fermentations. The transformants were screened using 50 ml cultivations and the mycelium for the RNA isolations was collected from 200 ml cultivations. The shake flask cultivations were grown for 7 days at 30°C, 250 rpm. The laboratory scale fermentor cultivations were performed for 5 days in 1 l Braun Biostat M fermentors (B. Braun).

### Example 4

### Cultivation of Nonomuraea flexuosa for enzyme production

*Nonomuraea flexuosa* DSM43186 (ATCC35864) was cultivated on rolled oats mineral medium plates (medium no. 84, Deutsche Sammlung von Microorganismen und Cellkulturen GmbH Catalogue of Strains, 1983) at 50°C. A sporulating colony was inoculated in XPYB medium, the GPYB medium (Greiner-Mei, et al., Syst. Appl. Microbiol., 9, 97-109, 1987) supplemented with 0.5% oat spelt xylan (Sigma X-0627, Sigma-Aldrich Corp., St. Louis, Mo.) instead of glucose as described in Holtz et al. (Antonie Leeuwenhoek, 59, 1-7, 1991) and was incubated in shake flask for 2 or 3 days (250 rpm, 50-55°C) after which the shake flask culture was used as a seed culture for the fermentation. The laboratory scale fermentor cultivations were performed for 3 days at 50°C in 1 liter Braun Biostat M fermentors (B. Braun, Melsungen AG, Melsungen, Germany) in the medium described above.

### Example 5

### Heterologous production of the thermostable Nonomuraea flexuosa AM35 xylanase in Trichoderma

The gene coding for the Nf Xyn11A (AM35) xylanase (*am35* or *Nf xyn11A;* EMBL accession no AJ508952) was isolated from a lambda ZAP Express® library prepared from partially digested (*Sau*3Λ) and size-fractionated *Actinomadura* ( *Nonomuraea*) *flexuosa* DSM43186 (ATCC35864) chromosomal DNA as described in US 6,300,113. The nucleotide sequence of the gene and the deduced amino acid sequence are shown in Fig. 1.

The *T*. *reesei* transformant strain ALKO4396 producing recombinant Nf Xyn11A was constructed by transforming the expression cassette pALK945 (Fig. 3) to *T*. *reesei* ALK03620. The *am35* gene is expressed from the *cbh1* promoter, as a fusion to a carrier polypeptide encoded by *man5A* core/hinge sequence. The construction of the plasmid pALK945 and the strain were performed as described in Paloheimo, et al., Appl. Environ. Microbiol., 69, 7073-7082, 2003).

*T. reesei* transformant strain ALKO4396 was sporulated on Potato Dextrose Broth (PD) agar slants (Difco, Detroit, Mis.) at 30°C. For enzyme production in laboratory scale fermentor, the compex lactose based cellulose inducing medium (Joutsjoki *et al.,* Curr. Genet., **24**, 223-228, 1993) was used. Fermentations were performed for 5 days in 1 l Braun Biostat M fermentors.

In addition to the full-length protein, the recombinant *T. reesei* ALKO4396 culture medium was observed, in Western blot, to contain shorter forms of Xyn11A and low amounts of unprocessed Man5A-Xyn11A fusion protein (Fig. 4A).

### Example 6

### Purification of N. flexuosa Xyn11A and the recombinant Xyn11A polypeptides.

Purification of the native Nf Xyn11A xylanase from the culture media of *N. flexuosa* DSM43186 and the recombinant Xyn11A xylanases from *T*. *reesei* ALKO4396 was performed by combining ion exchange chromatography, hydrophobic interaction chromatography (HIC) and gel filtration in the following way. The growth medium of 11 fermentation was centrifuged at 8,000 x g for 30 min at 4°C. The supernatant was adjusted to pH 9.1 with 1 M NaOH and diluted with distilled water (until conductivity 4 mS/cm). This sample was applied to a DEAE Sepharose Fast Flow (Amersham Biosciences AB, Uppsala, Sweden) ion -exchanger (SR column, 5x15,5 cm diameter, 300 ml) equilibrated with 20 mM Na₂HPO₄ (pH 9.1) using a Fast Protein Liquid Chromatography (FPLC) system (Amersham Biosciences) at 4°C. Flow rate was 20 ml/min. Column was washed with 400 ml of 20 mM Na₂HPO₄ (pH 9.1).

Flow-through proteins were collected into 100 ml fractions. Elution of the bound proteins from the DEAE-column was accomplished by a linear gradient from 20 mM Na₂HPO₄ (pH 9.1) to 20 mM Na₂HPO₄ (pH 9.1) containing 0.5 M NaCl at 20 ml/min for 30 min, 5 ml fractions were collected. Finally column was washed with 300 ml of 20 mM Na₂HPO₄ (pH 9.1) containing 0.5 M NaCl. Fractions were analysed for xylanase activity and purity of proteins was assessed by SDS-PAGE and Western blots.

The flow-through fractions containing xylanase activity were pooled (up to 500-600 ml) and adjusted to contain 2 M NaCl and applied to a Phenyl Sepharose 6 Fast Flow (Amersham Biosciences) column (XR50/30 column, 5x11 cm, 215 ml) equilibrated with 40 mM Na₂HPO₄ (pH 9.1) containing 2 M NaCl. After washing with 400 ml of 40 mM Na₂HPO₄ (pH 9.1) containing 2 M NaCl elution was performed at 20 ml/min with a two-step gradient. First the proteins were separated using linear gradient (400 ml) of decreasing NaCl concentration (2-0 M) in 40 mM Na₂HPO₄ (pH 9.1). After washing with 200 ml of 40 mM Na₂HPO₄ (pH 9.1) the proteins were eluted with an increasing gradient (600 ml) of 0-60% ethylene glycol in 40 mM Na₂HPO₄ (pH 9.1). Finally the column was washed with 200 ml of 60% ethylene glycol in 40 mM Na₂HPO₄ (pH 9.1). 10 ml fractions were collected and analysed for xylanase activity and purity.

Pooled HIC-fractions containing xylanases of different molecular masses were concentrated on an Amicon ultrafiltration unit (Millipore Corp., Billerica, Mass.) with Diaflo® (PM10 62mm 10 PK) membrane, cut-off 10 kDa. Concentrated sample (10 ml) was subjected to gel exclusion chromatography on a HiLoad 26/60 Superdex 75 prep grade column (2,6x61 cm, 320 ml) equilibrated with 40 mM Na₂HPO₄ (pH 9.1) containing 0.1 M NaCl. Elution was performed with 400 ml of 40 mM Na₂HPO₄ (pH 9.1) containing 0.1 M NaCl. 6 ml fractions were analysed for xylanase activity purity.

With *N. flexuosa* culture medium roughly half of the xylanase activity loaded on the DEAE Sepharose FF column was found in the flow-through and half was bound to the DEAE column. With *T*. *reesei* culture medium the xylanase activity was in the flow-through. Most of the native *T*. *reesei* proteins, e.g. cellulases and the mannanase core/hinge region used as a fusion partner were bound to the DEAE column.

The flow-through fractions containing xylanase activity were pooled for the HIC run. After the Phenyl Sepharose 6 FF column three different forms of the recombinant Xyn11A xylanase were separated (Fig. 4B). The 37 kDa form (on SDS-PAGE, lane 3) corresponds to the native Nf Xyn11A purified from the *N. flexuosa* culture medium. The shorter forms had molecular masses of 30 kDa (lane 4) and 27 kDa (lane 5) on SDS-PAGE. These shorter forms eluted from the Phenyl Sepharose 6 FF column with 0 M NaCl, first the 30 kDa form and the 27 kDa form in subsequent fractions. The 37 kDa polypeptide eluted at 15-30% ethylene glycol concentration. The 30 kDa xylanase was further purified using Superdex 75 gel filtration.

### Example 7

### Characterization of the native Xyn11A and the recombinant Xyn11A polypeptides

Characteristics of the native Nf Xyn11A and the three recombinant Xyn11A polypeptides are presented in Table 1. The molecular masses estimated from the SDS-PAGE were signicantly higher than deduced from the amino acid sequence or determined by analysing the protein with the mass spectrometer. The molecular mass of the native Nf Xyn11A was determined to be 32857 Da, corresponding the calculated molecular weight (32876 Da) of the mature enzyme. It represents the full-length protein consisting of the catalytic domain and the CBM separated by the linker region. The molecular mass of the recombinant full-length Xyn11A was 33429 Da, which is 572 Da higher than that of the native Nf Xyn11A and 553 Da higher than the calculated value. The molecular masses of the 30 kDa and 27 kDa polypeptides were determined to be 23769 Da and 21974 Da, respectively. The recombinant Xyn11A polypeptides were named as r33.4 kDa, r23.8 kDa and r22.0 kDa polypeptides (Table 1) on the basis of their molecular masses on mass spectrometry. The N-terminus of all four polypeptides was DTTITQ (SEQ ID NO:20) which suggests different C-terminal processing in the r23.8 kDa and r22.0 kDa polypeptides. The C-terminal processing sites are shown in Fig. 1.

The specific activities of the Nf Xyn11A and the recombinant Xyn11A polypeptides were similar on birch xylan substrate (15568-17367 nkat/mg) (Table 1). Also, the pIs of the full-length proteins, Nf Xyn11A and the r33.4 kDa polypeptide were very similar (8.5 vs. 8.6), but they both differed from the value calculated from the amino acid sequence (7.9). The pIs of the r23.8 kDa and r22.0 kDa polypeptides lacking the CBMs were lower than those of the full-length enzymes (7.6 and 8.2 vs. 8.5-8.6).

### Example 8

### Temperature and pH dependences of the purified Xyn11A xylanases

The pH and temperature optima for xylanase activity were determined by incubating the Xyn11A samples at different pH values (pH 5-8) at temperatures from 60°C to 80°C for 60 min. Both at pH 5 (Fig. 5A) and pH 6 (data not shown) the maximal activity was reached at 80°C, and at pH 7 at 70°C (Fig. 5B). At all pHs the native Nf Xyn11A was the most thermophilic. This was seen especially at pH 8, where the optimum for Nf Xyn11A was at 70°C, and for the recombinant Xyn11A polypeptides at 60°C (Fig. 5C). The thermostability of the enzymes was determined in the absence of BSA. At 70°C no reduction in enzyme activity was found even after several hours of incubation. At 80°C the reduction was dependent of pH and the enzyme form. At pH 5 the r22.0 kDa and r23.8 kDa polypeptides were more stable (half-lives of 123 min and 157 min) than the full-length Xyn11A polypeptides, r33.4 kDa and Nf Xyn11A (half-lives of 13 min and 32 min) (Fig. 6; Table 1). The enzymes behave similarly also at pH 7, although the half-lives were shorter, 63-95 min for the r22.0 kDa and r23.8 kDa polypeptides, and 17-31 min for the full-length enzymes.

### Example 9

### Bleaching experiments using the purified Xyn11A forms

The *N. flexuosa* cultivation medium, the purified native Xyn11A and the recombinant Xyn11A polypeptides produced in *T. reesei* were tested in a single-stage peroxide bleaching with Finnish oxygen-delignified softwood kraft pulp (starting brightness 34% ISO, kappa number 20 and dry matter content 29.9%) at 100 nkat/g pulp dry matter *N. flexuosa* supernatant also 50 nkat/ml). The purified enzymes were supplemented with *T*. *reesei* culture medium to stabilize the enzymes. The amount of culture medium corresponded to the ratio of thermoxylanase activity and total protein content in the original recombinant *T*. *reesei* culture media used for enzyme purifications. After these additions the mixtures resembled the enzyme preparations to be used in actual industrial applications. Pulp treatments were carried out at 3% consistency at 80°C and pH 8 for one hour. Reference pulp was treated similarly without enzyme addition. After enzyme treatments the pulp was washed with distilled water. Chelation was performed by adding EDTA to 0.2% of dry matter and carried out at 3.0% consistency at pH 5 for one hour. Pulp was bleached at 10% pulp consistency at 80°C for three hours (H₂O₂ 3%, NaOH 3%, diethylenetriamine pentaacetic acid 0.2%, MgSO₄ 0.5% by volume), after which pulp was acidified with H₂SO₄, washed with distilled water and made into paper handsheets.

Reducing sugars in the chelated pulp were analyzed by the dinitrosalicylic acid method. The quality of the bleached and washed pulps was analyzed by determining the kappa number according to the TAPPI Test Method T 236 and viscosity according to the SCAN28 Scandinavian method. Brightness of the handsheets was analysed according to ISO 2470. Peroxide consumption was determined by titration.

In the single-step peroxide bleaching experiment performed with the *N. flexuosa* cultivation medium lignin removal (0.7-1.1 Kappa units depending on enzyme dosage) and brightness increase (1.0 -1.1 ISO units) was obtained with no reduction in the pulp strength determined by viscosity (Table 2). The *T. reesei* culture medium increased the brightness by 0.9 ISO units. A further increase of 1.1-1.6 ISO units was obtained with the purified recombinant Xyn11A polypeptides, r33.4 kDa, r23.8 kDa and r22.0 kDa - the r22.0 kDa polypeptide being the least efficient (Table 2).

### Example 10

### Construction of the expression cassettes for heterologous production of Nf Xyn11A (AM35) and the truncated Nf Xyn11A (AM24) in T. reesei

For production of Nf Xyn11A (AM35) and the truncated Nf Xyn11A (AM24) in *T. reesei* several expression cassettes were constructed that either include a fungal signal sequence or the fungal signal sequence and a variable carrier polypeptide for these two proteins. The *am35* /*am35** (see below) and *am24*/*am24** (see below) genes were expressed from the *T*. *reesei cel7A* promoter. The expression cassettes constructed are listed in Table 3 and their general structure is shown in Fig. 6. The Table 3 also includes the other relevant information on the constructs. The promoter, transcription terminator and 3'-flanking sequences were as described in (Karhunen, et al., Mol. Gen. Genet., 241, 515-522, 1993). The gene coding for acetamidase (*amdS*) was used as a marker in the transformations. The *amdS* gene was isolated from p3SR2 (Kelly and Hynes, EMBO J., 4, 75-479, 1985). A 3.1 kb *Spe* I-*Xba*I fragment was ligated between the *cel7A* terminator and 3'-flanking region. In addition to these two genes, *am35* and *am24,* with the native codon usage, the following 9 changes were made to codons in some of the constructs (see Table 3), to make the codons more favorable to *T*. *reesei*: Gly₅₃ GGG to GGC, Ala₆₆ GCG to GCC, Gly₆₈ GGG to GGC, Arg₈₅ CGG to CGC, Gly₈₈ GGG to GGC, Gly₁₀₀ GGA to GGC, Arg₁₀₁ CGG to CGC, Arg₁₀₂ CGG to CGC and Val₁₀₄ GTG to GTC. The genes including the changes described above were designated as *am35** and *am24**. The changes made did not change the amino acid sequence encoded by the genes, compared to *am35* and *am24*.

When the full-length *N. flexuosa* Xyn11A was produced, the *am35* and *am35** genes were included in the expression cassettes either as a 1.3 kb fragment ending at the *Mlu*I site about 250 bps after the stop codon or as an exact fusion of the *am35* to the *cbh1* terminator. The shortened genes, *am24* and *am24** ended to nucleotide 1091 (Fig. 1) and they were exactly fused to *cbh1* terminator including the stop codon. All the genes were fused from the sequence encoding the N-terminal Asp₄₄ (from nucleotide 432, Fig. 1) to the *man5A* signal sequence (pALK1118 and pALK1276, *man5A* nucleotides 1-57) or to a sequence encoding a carrier polypeptide. The carrier polypeptide sequences included the *man5A* core/hinge encoding sequence (pALK1022, pALK1309, pALK1131 and pALK1692, 1-1359), the sequence encoding a fragment of the *man5A* core (pALK1264, pALK1151 and pALK1154, 1-681) and the *cel6A* CBD/hinge (blocks A and B in pALK1285 and pALK1502, 1-306). For the *man5A* sequence, see Stålbrand, et al., (Appl. Environ. Microbiol., 61, 1090-1097, 1995). For the *cel6A* sequence, see (Teeri, et al., Gene, 51, 43-52, 1987). A synthetic sequence coding for the dipeptide Lys - Arg, a target of a Kex2-like protease (Calmels, et al., J. Biotechnol., 17, 51-66, 1991) was included in the linkers of all the constructs including the carrier protein (not in the signal sequence constructs pALK1118 and pALK1276). In addition, the linkers of pALK1264, pALK1151 and pALK1154 were preceded by a sequence coding for the amino acids Gly - Gln - Cys - Gly - Gly (SEQ ID NO:22). This additional sequence was included to increase the length of the linker between this non-intact carrier and the recombinant xylanase. An identical sequence, naturally occurring in the Man5A polypeptide, is preceding the xylanase sequence in pALK1022.

Exact fusions between the *cel7A* promoter and the signal sequences, carrier, linker, *xyn11A* sequences and terminator were synthesized by PCR. An *Nru*I recognition site (TCGCGA (SEQ ID NO:24)) was introduced into the Kex2 linker (coded by a sequence CGC GAC AAG CGC (SEQ ID NO:25)) to facilitate the construction of the fusions. The codon CGC was chosen for the arginines in the linker and the third nucleotide of the native codon preceeding the linker was changed to T, when necessary. The modifications made did not change the amino acids encoded by the constructs.

### Example 11

### Transformation of Trichoderma and analysis of the transformants.

*T. reesei* protoplasts were transformed with linear expression cassettes isolated from the vector backbones by *Eco*RI. The expression cassettes were transformed to *T*. *reesei* strain ALK03620 (Ce15A⁻) and/or to ALKO4468 (Ce15A⁻, Ce17B⁻), see the Table 4. Transformations were performed as in Penttilä, et al. (Gene, 61, 155-164, 1987) with the modifications described in Karhunen, et al. (Mol. Gen. Genet., 241, 515-522, 1993). The transformants were purified on selection plates through single conidia prior to sporulating them on PD. Targeting to the *cel7A* locus was screened as Cel7A-negative phenotype using Minifold I-SRC 96 dot blotter (Schleicher & Schuell, Dassel, Germany). The monoclonal antibody CI-258 or CI-261 (Aho, et al., Eur. J. Biochem., 200, 643-649, 1991) was used in the detection of Ce17A protein by the ProtoBlot Western blot AP system (Promega). The genotypes of the chosen transformants were confirmed by using Southern blots in which several genomic digests were included and the respective expression cassettes were used as probes. Strains containing a replacement of the *cel7A* with one copy of the expression cassette were chosen for further studies.

### Example 12

### Production of the AM35 and AM24 xylanases by single-copy T. reesei transformants

We have previously shown (Paloheimo, et al., Appl. Environ. Microbiol., 69, 7073-7082, 2003) that when a carrier polypeptide with an intact domain structure was used, higher production level of AM35 was obtained in *T*. *reesei* compared to the constructs without a carrier or with a carrier of non-intact domain structure. Also, the recombinant AM35 proteins had the same thermostability as the xylanase activity in the *N. flexuosa* cultivation supernatant. The xylanase activities were stable for at least two hours at 70°C, pH 7. The culture supernatants were also found to increase the brightness of pulp in laboratory scale peroxide of kraft pulp at high temperature and pH in the same way as the culture supernatant from *N. flexuosa.*

Now, the expression cassettes contained either the *am35*/*am35** gene encoding the full-length Xyn11A or a shortened version from it, *am24*/*am24**, encoding the truncated Xyn11A protein. Both these proteins were produced using three different carrier polypeptides and also without a carrier polypeptide (only a fungal signal sequence was included). The carriers had either an intact domain structure as Man5A core/hinge of Cel6A CBD/hinge or a non-intact domain structure as Man5A core/hinge fragment.

The expression cassettes shown in Table 3 (Fig. 6) were isolated from the expression plasmids and transformed to *T. reesei* ALKO3620 and/or ALKO4468. The transformants containing single-copy replacement of *cel7A* gene by the expression cassettes were screened for further analysis. Several parallel single-copy strains from each construct were similar in terms of protein and xylanase activity levels analyzed from the culture supernatants of shake flask cultivations. One single-copy representative from each construct was chosen to be cultivated in the fermentor. The culture supernatants from the fermentor cultivations were analysed for the amount of protein and xylanase activities (Table 4).

The results from fermentor cultivations (Table 4) showed that the best xylanase activities were obtained from the *T*. *reesei* transformants including the constructs in which the shortened genes, *am24* or *am24**, were used. The increase in xylanase activity was observed with all the carriers tested and also when no carrier protein was included (e.g. RF5024 vs. RF5724, RF5725 vs. ALKO4405, RF5139 vs. RF5013, RF4861 vs. ALKO4823). The changes made to codons did not have an effect on the xylanase production level (ALKO4405 vs. RF5510 and RF4861 vs. RF4878). Also, similar levels of activity were obtained from the transformant with the construct in which the xylanase gene was fused exactly to the terminator sequence compared to the transformant including the construct in which there was a non-exact fusion of the xylanase gene to the terminator (RF5745 vs. ALKO4405) This similar level of activity was obtained even though, for unknown reason, the level of total protein in the culture supernatant of RF5745 including an exact terminator fusion was lower than in the corresponding transformant with a non-exact terminator fusion. Both the *T*. *reesei* host strains used, transformed with the same expression cassette did produce similar amount of activity (RF5725 vs. ALKO4812).

Because the specific activities of the AM35 and AM24 proteins are very similar to each other (Table 1, r33.4 and r23.8 kDa) it can be concluded that shortening of *Nonomuraea* xylanase gene increases the production level of xylanase in *T*. *reesei.* When the carriers with an intact domain structure were used (Man5A core/hinge and Cel6A CBD/hinge), the xylanase activity levels measured from the culture supernatants were over three-fold higher for the constructs in which AM24 was produced compared to the corresponding constructs for AM35. For the constructs in which no carrier polypeptide was included or the carrier was a fragment of Man5A (no intact domain structure), the increase in xylanase activity in the culture supernatant was even higher, from over 5- to over 10-fold. Thus, even with no carrier or by using carrier having a non-optimal structure, the yield of the bacterial xylanase could be increased to a surprisingly high level when a shortened xylanase gene was expressed in *T*. *reesei.*

Increases in the levels of xylanase activity were also observed in the shake flaks cultivations. When no carrier polypeptide was used, the increase in xylanase activity (RF5024 compared to RF5724) was about 2.8-fold and reached 4900 nkat/ml. When Man5A core/hinge was used as a carrier (RF5725 compared to ALKO4405 the increase in activity was about 2.7-fold (RF5725 produced about 21 000 nkat/ml). By using the Cel6A CBD carrier (RF5139 vs. RF5013) the increase in activity was even higher, about 5.6-fold. This high increase was due to the low level of activity in shake flasks from the strains producing AM35 with the Cel6A CBD carrier polypeptide (e.g. RF5013 produced about 3000 nkat/ml, and the activity from RF5139 cultivations was about 16 800 nkat/ml).

The Fig. 8 shows the AM24 xylanase product from a *T*. *reesei* transformant (Ce16A CBD carrier was used). In addition to the protein having the expected molecular mass, also a xylanase form having a lower molecular mass (corresponding to r22.0 kDa) can be detected from the culture supernatant. This form is due to a proteolytic cleavage of the AM24 protein.

### Example 13

### The use of the T. reesei culture supernatants containing the recombinant truncated Nf Xyn11A (AM24) xylanase in bleaching

Culture supernatant from a *T*. *reesei* transformant producing the recombinant truncated Xyn11A protein (AM24) was tested in bleaching of kraft pulp (Scandinavian birch and pine). The results obtained are shown on Tables 5 and 6. The same brightness could be obtained with lower ClO₂ consumption in both the bleaching experiments when AM24 was used compared to the reference bleaching without a treatment with the xylanase preparation.

### Example 14

### The use of the T. reesei culture supernatants containing the recombinant AM24 xylanase in feed application

Two feeding trials with broilers were performed with the AM24 xylanase preparation. The results from the trials demonstrated a good effect of AM24 in poultry. The body-weight in wheat-barley based diet was increased, compared to un-supplemented control birds, by 3.4% and in wheat and soy bean meal by 3.1 - 3.7%. Also, nutrient digestibility was significantly increased as measured by feed conversion rate. The AM24 xylanase preparation was at least as effective as the *T. reesei* XYNII (XYLII) used for years in animal nutrition. The advantage of the AM24 xylanase is the high heat stability compared to e.g. *T*. *reesei* XYNII (XYLII) which is a great improvement for several feed production procedures.

### Example 15

### The production of Nf Xyn10A (AM50) and two truncated forms of Nf Xyn10A in T. reesei

The gene coding for the Nf Xyn10A (AM50) xylanase (*am50* or *Nfxyn10A;* EMBL accession no AJ508953) was isolated from a lambda ZAP Express® library prepared from partially digested (*Sau*3A) and size-fractionated *Actinomadura* ( *Nonomuraea) flexuosa* DSM43186 (ATCC35864) chromosomal DNA as described in US 6,300,113. The nucleotide sequence of the gene and the deduced amino acid sequence are shown in Fig. 2. Three expression cassettes (Fig. 9) are constructed for production of the full-length AM50 protein (amino acids A₄₅ - A₄₉₂ from Fig. 2) and two truncated forms from it, the core (A₄₅ - N₃₄₅) and the core/linker missing all the three subdomains from the tail (A₄₅ - 5₃₆₇). Also, constructs could be made that encode Xyn10A polypeptides deficient in only the γ-subdomain or the γ- and β-subdomains of the tail. The estimated molecular masses of the different polypeptides would be (from the N-terminal A₄₅, without any added sugar moieties): core 34.0 kDa, core-linker 35.9 kDa, core-linker-α-subdomain of the tail 40.6 kDa, core-linker-a+β-subdomains of the tail 44.8 kDa, core-linker-α+β+γ-subdomains of the tail (full length mature protein) 49.1 kDa. The standard molecular biology methods, PCR reactions and annealing of oligonucleotides are used to make exact fusions between different sequences encoding: the *cbh1* promoter, *cel6A* signal sequence and sequence encoding Cel6A CBD (A+B), Kex2 site (RDKR), sequence encoding *Nfxyn10A* (core or core/hinge or the full-length protein) and *cbh1* terminator. The restriction sites at the end and the beginning of the fragments to be fused are included in the oligonucleotides to enable easy construction of the fusions. Finally, the *amdS* marker gene and the *cbh1* 3' -flanking region will be included as in the constructs made for expressing the *am35*/*am24* genes. The expression cassettes are isolated from the vector backbones using *EcoRI* digestion and they are transformed to *T. reesei* host strain. The same methods for the transformation, handling and selection of the transformants will be used as in construction of the strains producing Nf Xyn11A the transformants are purified through single conidia and they are screened in shake flask cultivations by measuring the xylanase activity from the cultivation supernatants. The single-copy transformants in which the expression cassette is replacing the *cbh1* locus will be chosen basing on the results on xylanase production level and Southern blot analysis of the genomes. The increased xylanase production from the strains producing the truncated forms of the Nf Xyn10A protein, compared to the strains producing the full length Nf Xyn10A is shown by using activity assays, SDS-PAGE and Western blot methods.

The culture supernatant(s) are used in the application tests, both for the bleaching of kraft pulp and feed applications, to show the effect of the xylanase(s).

**TABLE 1. Characteristics of the purified Nf Xyn11A and the recombinant Xyn11A polypeptides.**

| Enzyme | MW SDS-PAGE | MW Mass spect | N-terminus | Sp. act. (nkat/mg) | pI | Half-life 80°C, pH (min) | Half-life 5 80°C, pH 7 (min) |
|---|---|---|---|---|---|---|---|
| Nf Xyn11A Recombinant enzymes | 37 | 32.9 | DTTITQ | 15 568 | 8.5 | 32 | 31 |
| | | | | | | | |
| r33.4 kDa | 37 | 33.4 | DTTITQ | 16 005 | 8.6 | 13 | 17 |
| r23.8 kDa | 30 | 23.8 | DTTITQ | 17 367 | 7.6 | 157 | 95 |
| r22.0 kDa | 27 | 22.0 | DTTITQ | 16 950 | 8.2 | 123 | 63 |

**TABLE 2. Peroxide bleaching with the N. flexuosa cultivation medium and the purified recombinant Xyn11A polypeptides.**

| Xylanase | Brightness/ Brightness increase (ISO) | Kappa | Viscosity (dm³/kg) | Reducing sugars (% of dry matter) | Peroxide consumption (%) |
|---|---|---|---|---|---|
| *N. flexuosa* medium^{a} | 72.9 | 8.3 | 890 | ND | ND |
| *N. flexuosa* medium^{b} | 73.0 | 7.9 | 890 | ND | ND |
| Reference | 71.9 | 9.0 | 870 | ND | ND |
| | | | | | |
| r33.4 kDa | 64.5/+2.4 | 8.8 | ND | 0.30 | 2.3 |
| r23.8 kDa | 64.6/+2.5 | 8.1 | ND | 0.31 | 2.3 |
| r22.0 kDa | 64.1/+2.0 | 8.7 | ND | 0.30 | 2.3 |
| *T. reesei* medium | 63.0/+0.9 | 9.2 | ND | 0.25 | 2.3 |
| Reference | 62.1 | 8.7 | ND | 0.25 | 2.2 |

| | | | | | |
|---|---|---|---|---|---|
| ^{a}Enzyme dosage 50 nkat/g pulp dry matter. ^{b}Enzyme dosage 100 nkat/g pulp dry matter. | | | | | |

**TABLE 3. The constructs used to express the N. flexuosa am35/am35* gene and the shortened am35/am35* gene (am24/am24*).**

| **Expr. cassette** | **Carrier protein** | **Xylanase gene** | **Term. fusion** | **Reference** |
|---|---|---|---|---|
| pALK1118 | No carrier (*man5A* ss) | *am35* | Non-exact | Paloheimo *et al.* (2003) |
| pALK1276 | No carrier (*man5A* ss) | *am24* | Exact | This application |
| pALK1022 | Man5A core/hinge | *am35* | Non-exact | Paloheimo *et al.* (2003) |
| pALK1692 | Man5A core/hinge | *am35* | Exact | This application |
| pALK1309 | Man5A core/hinge | *am35** | Non-exact | This application |
| pALK1131 | Man5A core/hinge | *am24** | Exact | This application |
| pALK1285 | Cel6A CBD/hinge | *am35* | Non-exact | Paloheimo *et al.* (2003) |
| pALK1502 | Cel6A CBD/hinge | *am24** | Exact | This application |
| pALK1264 | Man5A fragment | *am35* | Non-exact | Paloheimo *et al.* (2003) |
| pALK1151 | Man5A fragment | *am24* | Exact | This application |
| pALK1154 | Man5A fragment | *am24** | Exact | This application |

**Table 4. Xylanase production from single-copy Trichoderma reesei transformants in laboratory scale fermentations.**

| **Strain** | **Expression cassette** | ***T. reesei* host** | **protein (mg/ml)** | **Xylanase (nkat/ml)** |
|---|---|---|---|---|
| **1. *T. reesei* host strains** | | | | |
| ALKO3620 | No cassette | | 14.4 | 1 490 |
| ALKO4468 | No cassette | | 9.3 | 430 |
| | | | | |

| **2. No carrier (Man5a signal sequence):** | | | | |
|---|---|---|---|---|
| ALKO4766 | pALK1118 | ALKO4468 | 8.8 | 2460 |
| RF5724 | pALK1118 | ALKO3620 | 11.3 | 3870 |
| RF5024 | pALK1276 | ALKO3620 | 7.7 | 31830 |
| | | | | |

| **3. Man5A core/hinge as a carrier** | | | | |
|---|---|---|---|---|
| ALKO4405 | pALK1022 | ALKO3620 | 12.3 | 14 400 |
| RF5745 | pALK1692 | ALKO3620 | 4.3 | 13 800 |
| RF5510 | pALK1309 | ALKO3620 | 11.9 | 14 600 |
| RF5725 | pALK1131 | ALKO3620 | 14.1 | 45940 |
| ALKO4812 | pALK1131 | ALKO4468 | 12.3 | 42980 |
| | | | | |

| **4. Cel6A CBD (A+B) as a carrier** | | | | |
|---|---|---|---|---|
| RF5013 | pALK1285 | ALKO3620 | 7.5 | 10750 |
| RF5139 | pALK1502 | ALKO3620 | 8.8 | 37140 |
| | | | | |

| **5. Fragment of Man5A as a carrier** | | | | |
|---|---|---|---|---|
| ALKO4823 | pALK1264 | ALKO4468 | 8.8 | 4940 |
| RF4861 | pALK1151 | ALKO3620 | 7.8 | 26400 |
| RF4878 | pALK1154 | ALKO3620 | 9.8 | 29600 |

**Table 5. Thermoxylanase in bleaching of birch mill kraft pulp**

| The sequence used was X/D-EO-D, the kappa number of the original birch kraft pulp used (from handsheet) was 14.4 and brightness 44.1%. The activity of the enzyme preparation used was 187 000 nkat/ml (measured at pH 7, 70°C using 5 min reaction time). ND = not determined. All the pH values were measured from pulp slurry at the reaction temperature. The pulp samples were acidified to pH 4.5 with SO₂-water before the sheet preparation to eliminate the residual chemicals. The pulp properties were tested according to ISO standard methods: brightness ISO 2470 (split pulp sheet), kappa numer ISO 302. tp = ton of pulp. aCl = active chlorine. | | | | | | |
|---|---|---|---|---|---|---|
| **Bleaching no** | | **1717** Ref. | **1718** | **1719** | **1720** | **1721** |
| **X-stage** | **Enzyme** | | | | | |
| Consist. 8% | Enzyme dosage, l/tp | no | 0.3 | 0.15 | 0.3 | 0.15 |
| Temp. 82°C | H₂SO₄, kg/tp (for pH adjustment) | 6.9 | 7.3 | 7.3 | 9.9 | 9.9 |
| Time 20 min | pH start / final | 7.0/7.6 | 7.0/7.9 | 7.0/7.9 | 6.0/6.3 | 6.0/6.3 |
| | | no washing after enzyme treatment | | | | |

| **D0-stage** | | | | | | |
|---|---|---|---|---|---|---|
| Consist. 9% | ClO₂ dosage, aCl kg/tp | 47.0 | 41.0 | 41.0 | 41.0 | 41.0 |
| Temp. 70°C | ClO₂ consumpt., aCl kg/tp | 47.0 | 41.0 | 41.0 | 41.0 | 41.0 |
| Time 30 min | final pH | 2.8 | 2.6 | 3.0 | 2.4 | 2.5 |
| **(EO)-stage** | NaOH dosage, kg/t | 21.0 | 21.0 | 21.0 | 21.0 | 21.0 |
| Consist. 10% | final pH | 10.3 | 10.2 | 10.3 | 10.3 | 10.3 |
| Temp. 85°C | **Brightness, %** | **79.3** | **79.1** | **78.9** | **80.3** | **79.6** |
| Time 60 min | Kappa number | 2.5 | 2.5 | 2.9 | 2.4 | 2.6 |
| 4 bar O₂ | XD(EO) yield, % | 93.4 | 93.2 | 93.9 | 91.3 | 92.5 |
| **D2-stage** | ClO₂ dosage, aCl kg/tp | 17.0 | 15.0 | 15.0 | 14.0 | 14.5 |
| Consist 9% | ClO₂ consumpt., aCl kg/tp | 15.6 | 14.4 | 14.4 | 13.2 | 13.2 |
| Temp 75°C | NaOH kg/tp (for pH adjustment) | 2.5 | 2.2 | 2.2 | 2.1 | 2.1 |
| Time 110 min | final pH | 4.3 | 4.6 | 4.6 | 4.3 | 4.3 |
| | **Brightness, %** | **90.1** | **90.2** | **90.1** | **90.7** | **903** |
| | Kappa number | 0.6 | 0.6 | ND | ND | ND |
| | D2-yield, % | 98.7 | 98.6 | 97.8 | 99.0 | 98.5 |
| | Total bleaching yield, % | 92.2 | 91.9 | 91.8 | 90.4 | 91.1 |
| | Tot. ClO₂ dosage, aCl kg/tp | 64.0 | 56.0 | 56.0 | 55.0 | 55.5 |
| | Tot. ClO₂ consumpt, aCl kg/tp | 62.6 | 55.4 | 55.4 | 54.2 | 54.2 |
| | Tot. NaOH kg/tp | 23.5 | 23.2 | 23.2 | 23.1 | 23.1 |
| | Tot. H₂SO₄, kg/tp | 6.9 | 7.3 | 7.3 | 9.9 | 9.9 |

**Table 6. Thermoxylanase in bleaching of softwood kraft pulp. Sequence X/D-E-D-E-D was used. The Kappa number of the original pulp was 28.9 (from handsheet), brightness 28.2% and viscosity 1180 ml/g. The activity of the enzyme preparation used was 187 000 nkat/ml (measured at pH 7, 70°C, 5 min reaction time). ND = not done. All the pH values were measured from pulp slurry at the reaction temperature. The pulp samples were acidified to pH 4.5 with SO₂-water before the sheet preparation to eliminate the residual chemicals. The pulp properties were tested according to ISO standard methods: brightness ISO 2470 (split pulp sheet), kappa numer ISO 302, viscosity ISO 5351/1. tp = ton of pulp. aCl = active chlorine.**

| **Bleaching no** | | **1627** Ref. | **1692** | **1698** |
|---|---|---|---|---|
| **X-stage** | **Enzyme** | | | |
| Consist. 8% | Enzyme dosage, 1/tp | no | 0.3 | 0.3 |
| Temp. 70°C | H₂SO₄, kg/tp (for pH adjustment) | 2.7 | 2.7 | 2.7 |
| Time 60 min | pH start / final | 7.0/7.4 | 7.0/7.3 | 7.0/7.3 |
| | | | | |
| | | | | |

| **D0-stagc** | | | | |
|---|---|---|---|---|
| Consist. 9% | ClO₂ dosage, aCl kg/tp | 60.0 | 54.0 | 54.0 |
| Temp. 50°C | ClO₂ consumpt., aCl kg/tp | 60.0 | 54.0 | 54.0 |
| Time 45 min | final pH | 1.7 | 1.9 | 1.9 |
| | | | | |
| **E1-stage** | NaOH dosage, kg/t | 27.0 | 27.0 | 27.0 |
| Consist. 10% | final pH | 10.9 | 11.1 | 11.1 |
| Temp. 60°C | **Brightness, %** | **49.5** | **49.6** | **49.4** |
| Time 60 min | Kappa number | 6.1 | 6.2 | 6.5 |
| | | | | |
| **D1-stage** | ClO₂ dosage, aCl kg/tp | 24.5 | 20.0 | 20.0 |
| Consist 10% | ClO₂ consumpt., aCl kg/tp | 24.5 | 19.8 | 20.0 |
| Temp 70°C | NaOH kg/tp (for pH adjustment) | 3.4 | 3.0 | 2.7 |
| Time 240 min | final pH | 3.6 | 4.2 | 4.1 |
| | **Brightness,** % | **78.9** | ND | **77.2** |
| | Kappa number | 1.8 | ND | ND |
| | | | | |
| **E2-stage** | NaOH dosage, kg/t | 8.0 | 8.0 | 8.0 |
| Consist. 10% | final pH | 10.6 | 10.6 | 10.6 |
| Temp. 70°C | **Brightness,** % | ND | 77.5 | ND |
| Time 60 min | Kappa number | ND | 1.5 | ND |
| **D2-stage** | ClO₂ dosage, aCl kg/tp | 9.0 | 8.0 | 9.5 |
| Consist. 10% | ClO₂ consumpt., aCl kg/tp | 7.9 | 7.6 | 9.3 |
| Temp. 70°C | NaOH kg/tp (for pH adjustment) | 1.1 | 0.9 | 1.1 |
| Time 240 min | final pH | 4.6 | 4.4 | 4.2 |
| | **Brightness,** % | 90.0 | 89.4 | 89.9 |
| | Kappa number | 0.7 | 0.7 | 0.6 |
| | Viscosity ml/g | 1030.0 | ND | 1050.0 |
| | Tot. ClO₂, aCl kg/tp | 93.5 | 82.0 | 83.5 |
| | Tot. NaOH kg/tp | 39.5 | 38.9 | 38.8 |
| | Tot. H₂SO₄, kg/tp | 2.7 | 2.7 | 2.7 |

## Claims

1. A recombinant DNA method for obtaining in a filamentous fungus host a higher production level of *Nonomuraea flexuosa* thermostable xylan degrading enzyme NfXyn11A, which in its original full length state has a structure consisting of a catalytic module (CAT) and a carbohydrate binding module (CBM) separated by a linker region, **characterized in that** a DNA construct, which comprises regulatory sequences derived from filamentous fungi and a shortened DNA sequence encoding a truncated form of said full length NfXyn11A enzyme, which truncated form is the amino acid sequence of SEQ ID NO:12 and contains the catalytically active region of CAT, but lacks part of the CBM is introduced into filamentous fungal host and allowed to express and secrete said truncated NfXyn11A enzyme, under the control of said filamentous fungal derived regulatory sequences, which include a promoter sequence, a signal sequence with or without a carrier sequence, and a terminator sequence derived from a filamentous fungus.

2. The method according to claim 1, wherein the DNA sequences encoding the carrier polypeptide are derived from filamentous fungi and encode selected regions or domains of a secretable carbohydrate degrading enzyme.

3. The method according to claim 2, wherein the DNA sequence encodes the carrier polypeptide Man5A core or core/hinge region, or Cel6A carbohydrate binding domain (CBD) consisting of a A, A+B or A+B+B', wherein A is carbohydrate binding domain, B is a hinge region, and B' is a duplicated hinge region.

4. The method according to claim 1, wherein the promoter sequences comprise *Trichoderma* or *Aspergillus* promoters.

5. The method according to claim 4, wherein the *Trichoderma* promoter is *cel7A (cbhl)* promoter.

6. The method according to claim 1, wherein the higher production level obtained with said DNA construct encoding the truncated form of said NfXyn11A enzyme measured as an increase of activity from single copy transformants is higher than the production level obtained with another corresponding DNA construct, which is identical with said DNA construct except that it comprises a DNA sequence encoding the corresponding full length NfXyn11A enzyme.

7. The method according to claim 6, wherein the higher production level achieved with the DNA construct encoding the truncated form of the NfXyn11A enzyme is at least 2 times higher than the production level achieved with another corresponding DNA construct, which is identical with said DNA construct except that it comprises a DNA sequence encoding the corresponding full length NfXyn11A enzyme.

8. A DNA construct for obtaining in a filamentous fungus host a higher production level of *Nonomuraea flexuosa* thermostable xylan degrading enzyme NfXyn11A having in its original full length state a catalytic module (CAT) and a carbohydrate binding module (CBM) separated by a linker region, **characterized in that** said DNA construct comprises regulatory sequences derived from filamentous fungi including a promoter, a signal sequence with or without a carrier sequence, and a terminator sequence and a shortened DNA sequence encoding a truncated form of said full length NfXyn11A enzyme, which truncated form is the amino acid sequence of SEQ ID NO:12 and contains the catalytically active region of CAT, but lacks part of the CBM.

9. The DNA construct according to claim 8, wherein the DNA sequences encoding the carrier polypeptide are DNA sequences derived from the same or different filamentous fungi and encode selected regions or domains of a secretable carbohydrate degrading enzyme.

10. The DNA construct according to claim 9, wherein the DNA sequence encodes the carrier polypeptide Man5A core or core/hinge region, or Cel6A CBD consisting of a A, A+B or A +B + B' region, wherein A is a carbohydrate binding domain, B is hinge domain and B' is a duplicated hinge domain.

11. The DNA construct according to claim 8, wherein the promoter sequences comprise *Trichoderma* or *Aspergillus* promoters.

12. The DNA construct according to claim 11, wherein the *Trichoderma* promoter is cel7A *(cbhl)* promoter.

13. The DNA construct according to claim 8, wherein the production level of said DNA construct encoding the truncated NfXyn11A enzyme measured as an increase of activity from single copy transformants is higher than the production level obtained with another corresponding DNA construct which is identical with said DNA construct, except that it comprises a DNA sequence encoding the corresponding full length NfXyn11A enzyme.

14. The DNA construct according to claim 13, wherein the higher production level achieved with said DNA construct encoding the truncated form of the NfXyn11A enzyme is at least two times higher than the production level achieved with another corresponding DNA construct, which is identical with said DNA construct, except that comprises a DNA sequence encoding the full length NfXyn11A enzyme.

## Patentansprüche

1. Rekombinantes DNA-Verfahren, um in einem filamentösen Wirtspilz eine höhere Produktion des *Nonomuraeaflexuosa* thermostabilen Xylan-abbauenden Enzyms NfXyn11A zu erzielen, welches in seinem ursprünglichen Volllängenzustand eine Struktur bestehend aus einem katalytischen Modul (CAT) und einem kohlenhydratbindenden Modul (CBM) getrennt durch eine Linkerregion hat,
**dadurch gekennzeichnet, dass** ein DNA-Konstrukt, umfassend von filamentösen Pilzen stammende regulatorische Sequenzen und eine gekürzte DNA-Sequenz kodierend für eine verkürzte Form dieses Volllängen-NfXyn11A-Enzyms, wobei die verkürzte Form die Aminosäuresequenz der SEQ ID NO:12 ist und die katalytisch aktive Region von CAT enthält, aber ihr ein Teil der CBM fehlt, in einen filamentösen Wirtspilz eingeführt wird und wobei es dem DNA-Konstrukt ermöglicht wird, dieses verkürzte NfXyn11A-Enzym zu exprimieren und sekretieren unter der Kontrolle der von dem filamentösen Pilz stammenden regulatorischen Sequenzen, welche eine Promotorsequenz, eine Signalsequenz mit oder ohne eine Trägersequenz und eine von einem filamentösen Pilz stammende Terminatorsequenz enthalten.

2. Verfahren nach Anspruch 1, worin die das Trägerpolypeptid kodierenden DNA-Sequenzen von filamentösen Pilzen stammen und ausgewählte Regionen oder Domänen eines sekretierbaren kohlenhydratabbauenden Enzyms kodieren.

3. Verfahren nach Anspruch 2, worin die DNA-Sequenz die Core- oder Core/Hinge-Region des Trägerpolypeptids Man5A oder die Ce16A kohlenhydratbindende Domäne (CBD) bestehend aus A, A+B oder A+B+B', worin A eine kohlenhydratbindende Domäne ist, B eine Hinge-Region ist und B' eine duplizierte Hinge-Region ist, kodiert.

4. Verfahren nach Anspruch 1, worin die Promotorsequenzen *Trichoderma-* oder Aspergillus-Promotoren umfassen.

5. Verfahren nach Anspruch 4, worin der *Trichoderma-Promotor* ein *ce17A (cbhl)* Promotor ist.

6. Verfahren nach Anspruch 1, worin die höhere Produktion, die mit diesem DNA-Konstrukt kodierend für die verkürzte Form jenes NfXyn11A-Enzyms erzielt wird, gemessen als eine Erhöhung der Aktivität von Einzelkopietransformanten höher ist als die Produktion, die mit einem anderen entsprechenden DNA-Konstrukt erzielt wird, welches identisch mit diesem DNA-Konstrukt ist, mit der Ausnahme, dass es eine DNA-Sequenz kodierend für das entsprechende Volllängen-NfXyn11A-Enzym umfasst.

7. Verfahren nach Anspruch 6, worin die höhere Produktion, die mit dem DNA-Konstrukt kodierend für die verkürzte Form des NfXyn11A-Enzyms erreicht wird, mindestens doppelt so hoch ist wie die Produktion, die mit einem anderen entsprechenden DNA-Konstrukt, welches identisch mit diesem DNA-Konstrukt ist, mit der Ausnahme, dass es eine DNA-Sequenz, kodierend für das entsprechende Vollängen-NfXyn11A-Enzym, umfasst.

8. DNA-Konstrukt, um in einem filamentösen Wirtspilz eine höhere Produktion des *Nonomuraea flexuosa* thermostabilen Xylan-abbauenden Enzyms NfXyn11A zu erzielen, das in seinem ursprünglichen Volllängenzustand ein katalytisches Modul (CAT) und ein kohlenhydratbindendes Modul (CBM) getrennt durch eine Linkerregion hat,
**dadurch gekennzeichnet, dass** dieses DNA-Konstrukt von filamentösen Pilzen stammende regulatorische Sequenzen enthaltend einen Promotor, eine Signalsequenz mit oder ohne einer Trägersequenz und eine Terminatorsequenz und eine gekürzte DNA-Sequenz kodierend für die verkürzte Form dieses Vollängen-NfXyn11A-Enzyms umfasst, wobei die verkürzte Form die Aminosäuresequenz der SEQ ID NO:12 ist und die katalytisch aktive Region von CAT enthält, aber ihr ein Teil der CBM fehlt.

9. DNA-Konstrukt nach Anspruch 8, worin die das Trägerpolypeptid kodierenden DNA-Sequenzen DNA-Sequenzen sind, die von gleichen oder unterschiedlichen filamentösen Pilzen stammen und ausgewählte Regionen oder Domänen eines sekretierbaren kohlenhydratabbauenden Enzyms kodieren.

10. DNA-Konstrukt nach Anspruch 9, worin die DNA-Sequenz die Trägerpolypeptid Man5A Core oder Core/Hinge-Region oder Ce16A CBD bestehend aus einer A, A+B oder A+B+B'-Region, worin A eine kohlenhydratbindende Domäne ist, B eine Hinge-Domäne und B' eine duplizierte Hinge-Domäne ist, kodiert.

11. DNA-Konstrukt nach Anspruch 8, worin die Promotorsequenzen *Trichoderma-* oder Aspergillus-Promotoren umfassen.

12. DNA-Konstrukt nach Anspruch 11, worin der *Trichoderma*-Promotor ein ce17A (*cbh1*)-Promotor ist.

13. DNA-Konstrukt nach Anspruch 8, worin die Produktion jenes DNA-Konstrukts kodierend für das verkürzte NfXyn11A-Enzym gemessen als eine Steigerung der Aktivität von Einzelkopietransformanten höher ist als die Produktion, die mit einem anderen entsprechenden DNA-Konstrukt erzielt wird, welches identisch mit diesem DNA-Konstrukt ist, mit der Ausnahme, dass es eine DNA-Sequenz kodierend für das entsprechende VolllängenNfXyn11A-Enzym umfasst.

14. DNA-Konstrukt nach Anspruch 13, worin die höhere Produktion, die mit diesem DNA-Konstrukt kodierend für die verkürzte Form des NfXyn11A-Enzyms erreicht wird, mindestens doppelt so hoch ist wie die Produktion, die mit einem anderen entsprechen DNA-Konstrukt erreicht wird, welches mit diesem DNA-Konstrukt identisch ist, mit der Ausnahme, dass es eine DNA-Sequenz umfasst, die das Vollängen-NfXyn11A-Enzym kodiert.

## Revendications

1. Procédé de l'ADN recombinant pour obtenir dans un champignon hôte filamenteux un niveau de production plus élevé de l'enzyme thermostable de *Nomomuraea flexuosa* dégradant le xylane, NfXyn11A, qui, dans son état de pleine longueur d'origine, possède une structure consistant en un module catalytique (CAT) et un module de liaison aux glucides (CBM) séparés par une région de liaison, **caractérisé en ce qu'**un produit de recombinaison d'ADN, qui comprend des séquences régulatrices dérivées de champignons filamenteux et une séquence d'ADN raccourcie codant pour une forme tronquée de ladite enzyme NfXyn11A de pleine longueur, laquelle forme tronquée est la séquence d'acides aminés de SEQ ID NO: 12 et contient la région catalytiquement active du CAT mais ne possède pas une partie du CBM, est introduit dans un champignon hôte filamenteux et peut exprimer et sécréter ladite enzyme NfXyn11A tronquée, sous le contrôle desdites séquences régulatrices dérivées d'un champignon filamenteux, qui comprennent une séquence promotrice, une séquence signal avec ou sans séquence porteuse, et une séquence de terminaison dérivées d'un champignon filamenteux.

2. Procédé selon la revendication 1, dans lequel les séquences d'ADN codant pour le polypeptide porteur sont dérivées de champignons filamenteux et codent pour des régions ou des domaines sélectionné(e)s d'une enzyme dégradant les glucides pouvant être sécrétée.

3. Procédé selon la revendication 2, dans lequel la séquence d'ADN code pour la partie centrale du polypeptide porteur Man5A et/ou une région centrale/charnière, ou le domaine de liaison aux glucides (CBD) de Ce16A consistant en A, A+B ou A+B+B', où A est un domaine de liaison aux glucides, B est une région charnière, et B' est une région charnière dupliquée.

4. Procédé selon la revendication 1, dans lequel les séquences promotrices comprennent des promoteurs de *Trichoderma* ou *Aspergillus.*

5. Procédé selon la revendication 4, dans lequel le promoteur de *Trichoderma* est le promoteur de *ce17A (cbhl).*

6. Procédé selon la revendication 1, dans lequel le niveau de production plus élevé obtenu avec ledit produit de recombinaison d'ADN codant pour la forme tronquée de ladite enzyme NfXyn11A, mesuré en tant qu'une augmentation de l'activité de transformants à une seule copie, est plus élevé que le niveau de production obtenu avec un autre produit de recombinaison d'ADN correspondant, qui est identique audit produit de recombinaison d'ADN sauf qu'il comprend une séquence d'ADN codant pour l'enzyme NfXyn11A de pleine longueur correspondante.

7. Procédé selon la revendication 6, dans lequel le niveau de production plus élevé obtenu avec le produit de recombinaison d'ADN codant pour la forme tronquée de l'enzyme NfXyn11A est au moins 2 fois plus élevé que le niveau de production obtenu avec un autre produit de recombinaison d'ADN correspondant, qui est identique audit produit de recombinaison d'ADN sauf qu'il comprend une séquence d'ADN codant pour l'enzyme NfXyn11A de pleine longueur correspondante.

8. Produit de recombinaison d'ADN pour obtenir dans un champignon hôte filamenteux un niveau de production plus élevé de l'enzyme thermostable de *Nomomuraea flexuosa* dégradant le xylane, NfXyn11A, possédant, dans son état de pleine longueur d'origine, un module catalytique (CAT) et un module de liaison aux glucides (CBM) séparés par une région de liaison, **caractérisé en ce que** ledit produit de recombinaison d'ADN comprend des séquences régulatrices dérivées de champignons filamenteux incluant un promoteur, une séquence signal avec ou sans séquence porteuse, et une séquence de terminaison et une séquence d'ADN raccourcie codant pour une forme tronquée de ladite enzyme NfXyn11A de pleine longueur, laquelle forme tronquée est la séquence d'acides aminés de SEQ ID NO: 12 et contient la région catalytiquement active du CAT mais ne possède pas une partie du CBM.

9. Produit de recombinaison d'ADN selon la revendication 8, dans lequel les séquences d'ADN codant pour le polypeptide porteur sont des séquences d'ADN dérivées de champignons filamenteux identiques ou différents et codent pour des régions ou des domaines sélectionné (e) s d'une enzyme dégradant les glucides pouvant être sécrétée.

10. Produit de recombinaison d'ADN selon la revendication 9, dans lequel la séquence d'ADN code pour la partie centrale du polypeptide porteur Man5A et/ou une région centrale/charnière, ou le CBD de Ce16A consistant en une région A, A+B ou A+B+B', où A est un domaine de liaison aux glucides, B est une région charnière et B' est une région charnière dupliquée.

11. Produit de recombinaison d'ADN selon la revendication 8, dans lequel les séquences promotrices comprennent des promoteurs de *Trichoderma* ou *Aspergillus.*

12. Produit de recombinaison d'ADN selon la revendication 11, dans lequel le promoteur de *Trichoderma* est le promoteur de ce17A (cbhl).

13. Produit de recombinaison d'ADN selon la revendication 8, dans lequel le niveau de production dudit produit de recombinaison d'ADN codant pour l'enzyme NfXyn11A tronquée, mesuré en tant qu'une augmentation de l'activité de transformants à une seule copie, est plus élevé que le niveau de production obtenu avec un autre produit de recombinaison d'ADN correspondant, qui est identique audit produit de recombinaison d'ADN sauf qu'il comprend une séquence d'ADN codant pour l'enzyme NfXyn11A de pleine longueur correspondante.

14. Produit de recombinaison d'ADN selon la revendication 13, dans lequel le niveau de production plus élevé obtenu avec ledit produit de recombinaison d'ADN codant pour la forme tronquée de l'enzyme NfXyn11A est au moins deux fois plus élevé que le niveau de production obtenu avec un autre produit de recombinaison d'ADN correspondant, qui est identique audit produit de recombinaison d'ADN sauf qu'il comprend une séquence d'ADN codant pour l'enzyme NfXyn11A de pleine longueur.
